# EUROPEAN PATENT SPECIFICATION

(11) **EP 2 126 060 B1**
(45) Date of publication and mention of the grant of the patent: **05.10.2016**
(21) Application number: 08717945.3
(22) Date of filing: 18.03.2008
(51) Int. Cl.: C12N 9/06, C12P 21/06, C12P 21/00, A23J 3/08, A23J 3/14, A23J 3/22

(54) **NOVEL LYSYL OXIDASES**
NEUE LYSYL-OXIDASEN
NOUVELLES LYSYL-OXYDASES

(30) Priority: 22.03.2007 EP 07104645; 12.12.2007 EP 07123028
(43) Date of publication of application: 02.12.2009
(73) Proprietor: DSM IP Assets B.V., 6411 TE Heerlen (NL)
(72) Inventor: DIJK, VAN, Albertus, Alard, 2600 Delft (NL); DEKKER, Petrus, Jacobus, Theodorus, NL-2497 AE Den Haag (NL); WESTERLAKEN, Ilja, NL-3032 GA Rotterdam (NL); BAKHUIS, Janna Gardina, NL-2627 AD Delft (NL)
(74) Representative: van Grieken-Plooster, Izabella Johanna
(86) International application number: PCT/EP2008/053212
(87) International publication number: WO 2008/113799

(56) References cited:
- EP-A- 0 988 859
- US-A1- 2005 142 650
- DATABASE UniProt Hypothetical protein 26 April 2005 (2005-04-26), XP002437126 retrieved from EBI Database accession no. Q5B038_EMENI cited in the application
- DATABASE UniProt Hypothetical protein 24 May 2005 (2005-05-24), XP002437127 retrieved from EBI Database accession no. Q55P44_CRYNE cited in the application
- DATABASE ENTREZ NCBI; Hypothetical protein 28 November 2006 (2006-11-28), XP002437128 retrieved from NCBI Database accession no. Q4HWI1 cited in the application
- DATABASE UniProt Hypothetical protein 1 June 2003 (2003-06-01), XP002437129 retrieved from EBI Database accession no. Q86ZN4_PODAN cited in the application

### SEQUENCE LISTING

<110> DSM IP Assets B.V.
<120> NOVEL LYSYL OXIDASES
<130> 25977WO
<160> 11
<170> PatentIn version 3.2
<210> 1
   <211> 817
   <212> PRT
   <213> Aspergillus nidulans ZGR
<400> 1
   Met Lys Leu Pro Leu Ser Phe Ser Gly Gly Ile Ala Leu Leu Leu Ile
   1               5                   10                  15
   Gly Ser Leu Ala Gly Asp Val Val Ala Thr Lys Leu Asp Pro Ser Arg
               20                  25                  30
   Asp Leu Val Arg Gln Lys Ser Arg Gly Arg Lys Asn Gln Met Arg Ser
           35                  40                  45
   Leu Val Gly Arg Ser Asn Gln His Ile Thr His Gln Ser Arg Pro Tyr
       50                  55                  60
   Thr Asn Glu Tyr Ala Ser Pro Cys Gln Ile Thr Pro Pro Gln Glu Ile
   65                  70                  75                  80
   Lys Ala Pro Lys Glu Asn Val Trp Tyr Gly Leu Thr Asp Asp Glu Thr
                   85                  90                  95
   Ala Asp Val Ala Lys Trp Leu Phe Gly Arg Pro Glu Leu Asn Leu Thr
               100                 105                 110
   Thr Thr Glu Asn Ala Gly Glu Trp Asp Asn Thr Ile Ala Leu Ile Glu
           115                 120                 125
   Leu His Arg Pro Asn Lys Ser Glu Ala Ile Pro Tyr Leu Asp Gly Ser
       130                 135                 140
   Gly Pro Ala Pro Thr Arg His Ala His Val Arg Leu Asn Asn Arg Ala
   145                 150                 155                 160
   Thr Thr Asp Pro Tyr Phe Ala Asp Ile Leu Val Gly Pro Leu Pro Val
                   165                 170                 175
   Ser Asn Ala Thr Thr Trp Glu Pro Leu Glu Phe Pro Tyr Thr Arg Lys
               180                 185                 190
   Thr Gln Gly Gln Val Arg Asn Val Glu Pro Asp Gly Glu Thr Val Tyr
           195                 200                 205
   Ser Glu Trp Leu Phe Lys Ile Ser Ala Ser Ile Ala Asp Ile Thr Leu
       210                 215                 220
   Asp Leu Trp Asn Gly Thr Ala Leu Gly Leu Glu Asn Asp Thr Leu Asp
   225                 230                 235                 240
   Ile Trp Gly Ile Asp Pro Leu Trp Gln Asp Asp Gly Arg Ile Ile Arg
                   245                 250                 255
   Trp Asp Met Phe Trp Asn Met Ala Asp Asp Glu Phe Asp Ser Glu Thr
               260                 265                 270
   Leu Leu Pro Leu Gly Leu Tyr Leu Lys Ser Asp Val Thr Gly Arg Asp
           275                 280                 285
   Pro Ser Gln Trp Lys Leu Leu Gly Trp Met Tyr Asn Asp Ile Phe Tyr
       290                 295                 300
   Glu Thr Thr Glu Glu Phe Arg Lys Ala Tyr Trp Ser Pro Gly Phe Val
   305                 310                 315                 320
   Lys Leu Lys Pro Asn Val Asp Gly Ala Trp Ala His Thr Glu Gln Arg
                   325                 330                 335
   Gly Pro Val Pro Pro Gln Asp Arg Lys Gln Pro Pro Val Met Ile Ala
               340                 345                 350
   Pro Asp Gly Ala Arg Tyr Ser Val Asp Ala Glu Arg Lys Tyr Val Thr
           355                 360                 365
   Trp Met Asp Phe Ser Phe Tyr Ile Ala Phe Asn Arg Asp Thr Gly Leu
       370                 375                 380
   Ser Leu Phe Asp Ile Lys Tyr Lys Gly Gln Arg Val Leu Tyr Glu Leu
   385                 390                 395                 400
   Gly Leu Gln Glu Ala Leu Ala His Tyr Ala Ala Asn Asp Pro Val Gln
                   405                 410                 415
   Ser Ser Val Ala Tyr Leu Asp Ser Tyr Tyr Gly Phe Gly Pro Tyr Ala
               420                 425                 430
   Phe Glu Leu Leu Lys Gly Tyr Asp Cys Pro Ser Tyr Ala Ser Tyr Leu
           435                 440                 445
   Asn Thr Ser Phe Tyr Lys Asp Glu Glu Thr His Thr His Val Asp Ser
       450                 455                 460
   Leu Cys Leu Phe Glu Phe Asp Ala Asp Tyr Pro Met Ala Arg His Ser
   465                 470                 475                 480
   Thr Ser Glu Phe Val Ser Val Thr Lys Asn Val Tyr Phe Thr Leu Arg
                   485                 490                 495
   Ser Val Ser Thr Ile Gly Asn Tyr Asp Tyr Met Phe Ser Tyr Asn Phe
               500                 505                 510
   His Met Asp Gly Thr Ile Gly Val Glu Val Arg Ala Ser Gly Tyr Ile
           515                 520                 525
   Gln Ser Ala Tyr Tyr Ala Asn Asn Gln Asp Phe Gly Tyr Gln Ile His
       530                 535                 540
   Asp Ser Leu Ser Gly Ser Met His Asp His Val Leu Asn Phe Lys Ala
   545                 550                 555                 560
   Asp Phe Asp Ile Leu Gly Pro Asn Asn Thr Ile Glu Leu Val Ser Val
                   565                 570                 575
   Val Pro Val Thr Lys Gln Phe Ser Trp Ser Gly Asn Lys Thr Arg Asn
               580                 585                 590
   Thr Met Gln Leu Gly Arg Ser Phe Ile His Ser Glu Asp Glu Ala Arg
           595                 600                 605
   Leu Asn Trp Gly Phe Asn Gly Gln Thr Gln Leu His Val Val Asn Gln
       610                 615                 620
   Asp Lys Pro Asn Lys Phe Gly Glu Pro Arg Gly Tyr Arg Ile Leu Pro
   625                 630                 635                 640
   Ser Ala Gly Thr Ala His Leu Thr Val Leu Asn Ser Ser Asn Leu Val
                   645                 650                 655
   His Ala Ala His Trp Ala Glu Tyr Asp Val Gln Val Thr Arg Gln His
               660                 665                 670
   Asp Phe Glu Pro Thr Ser Ala His Pro Tyr Asn Ser Gln Asp Ile His
           675                 680                 685
   Asn Pro Pro Val Asp Phe Ser Thr Phe Phe Asn Gly Glu Ser Leu Asn
       690                 695                 700
   Gln Thr Asp Leu Val Val Trp Leu Asn Leu Gly Met His His Val Pro
   705                 710                 715                 720
   His Thr Gly Asp Leu Pro Asn Thr Val Phe Thr Thr Ala His Ser Gly
                   725                 730                 735
   Val Ala Phe Thr Pro Leu Asn Tyr Leu Pro Gly Asp Pro Ser Arg Glu
               740                 745                 750
   Thr Val Asn Met Val Arg Val Asp Tyr Ser Asp Gly Ala Ala Thr Ala
           755                 760                 765
   Val Arg Thr Phe Gly Gln Ser Asn Glu Thr Cys Ser Val Val Leu Gln
       770                 775                 780
   Pro Val Glu Asn Glu Leu Trp Ser Tyr Gln Gly Asp Val Val Val Arg
   785                 790                 795                 800
   Lys Phe Pro Tyr Asp Pro Asn Asp Pro Phe Tyr Glu Thr Asp Ser Asp
                   805                 810                 815
   Ala
<210> 2
   <211> 801
   <212> PRT
   <213> Cryptococcus neoformans ZGT
<400> 2
   Met Arg Ala Ile Ala Leu Leu Tyr Leu Leu Gly Thr Ala Phe Ala Val
   1               5                   10                  15
   Pro Ser Pro Lys Ile Ser Asn Val Lys Lys Pro Gly His Tyr His His
               20                  25                  30
   Pro His Arg Arg Asp Ile Val Thr Asn Glu Val Asp Ala Ser Ala Ser
           35                  40                  45
   Ser Pro Thr Thr Ser Ala Pro Lys Asp Asn Ile Trp Asn Phe Leu Ser
       50                  55                  60
   Asn Asp Glu Ala Ala Gly Ile Ile Ala Phe Leu His Ser Gln Thr Glu
   65                  70                  75                  80
   Leu Asn Leu Thr Ala Val Asn Asp Ala Gly Asp Trp Asp Asn Thr Ile
                   85                  90                  95
   Thr Val Val Asp Leu Leu Pro Pro Asn Lys Thr Glu Ala Leu Ser Tyr
               100                 105                 110
   Met Asp Gly Asn Gly Thr Lys Pro Glu Arg Trp Gly Ile Ala Ser Leu
           115                 120                 125
   Leu Cys Gly Ala Thr Glu Glu Pro Tyr Ala Gln Asp Leu Val Val Gly
       130                 135                 140
   Pro Leu Pro Val Ser Glu Val Thr Ile Tyr Tyr Pro Tyr Thr Tyr Gly
   145                 150                 155                 160
   Thr His Ala Pro Asp Ala Lys Ile Arg Val Tyr Asp Met Asp Asp Asn
                   165                 170                 175
   Ser Glu Phe Leu Ser Asp Ile Ala Met Ser Met Lys Asp Ile Ile Ser
               180                 185                 190
   Asp Ile Leu Asn Ala Thr Ile Asp Thr Ala Asp Asp Leu Ala Asp Thr
           195                 200                 205
   Phe Asp Ile Trp Gly Ile Asp Pro Leu Trp His Gln Pro Asp Glu Asn
       210                 215                 220
   Gly Asn Asp Arg Val Ile Tyr Trp Ala Gly Phe Trp Arg Tyr Pro Asp
   225                 230                 235                 240
   Ser Leu Gln Met Glu Asn Ser Thr Ile Asn Phe Asp Gly Glu Thr Leu
                   245                 250                 255
   Leu Pro Gln Gly Leu Tyr Ile Gln Thr Asp Ile Thr Gly Arg Asp Arg
               260                 265                 270
   Ser Lys Trp Ala Leu Met Gly Ile Leu Tyr Gly Asp Asp Tyr Tyr Thr
           275                 280                 285
   Ser Val Asp Glu Phe Arg Ala Ala Trp Arg Lys Pro Gly Phe Lys Lys
       290                 295                 300
   Phe Thr Pro Asn Tyr Ser Gly Asp Trp Ile Gly Thr Asp Gln Thr Gly
   305                 310                 315                 320
   Asp Val Met Pro Phe Glu Thr Glu Ala Pro Pro Met Asn Val Gln Pro
                   325                 330                 335
   Gly Gly Gln Arg Phe Lys Val Asp Glu Asp Asn Lys Tyr Val Glu Trp
               340                 345                 350
   Met Asp Phe Ser Phe Tyr Leu Thr Phe Thr Arg Asp Thr Gly Met Arg
           355                 360                 365
   Leu Tyr Asp Val Lys Phe Lys Gly Glu Arg Ile Ile Tyr Glu Leu Gly
       370                 375                 380
   Leu Gln Glu Ala Ile Ala His Tyr Ala Gly Asn Asp Pro Val Gln Ser
   385                 390                 395                 400
   Gly Thr Ala Tyr Leu Asp Thr Tyr Tyr Gly Phe Gly Pro Tyr Ala Phe
                   405                 410                 415
   Ser Gln Val Pro Gly Phe Asp Met Pro Leu Tyr Ala Tyr Cys Met Asn
               420                 425                 430
   Thr Ser Phe His Ala Ala Glu Leu Ser Thr Ser His Arg Cys Gly Ile
           435                 440                 445
   Ser Ile Phe Glu Ala Asp Gln Asn Tyr Pro Ile Gln Arg His Ser Asn
       450                 455                 460
   Met Asn Tyr Val Ser Ala Thr Lys Asn Ile Ala Leu Thr Leu Arg Ser
   465                 470                 475                 480
   Ile Ser Thr Val Gly Asn Tyr Asp Tyr Asn Phe Asp Tyr Asn Phe Tyr
                   485                 490                 495
   Leu Asp Gly Thr Ile Glu Thr Val Val Arg Ala Ser Gly Tyr Ile Gln
               500                 505                 510
   Ser Ala Phe Tyr Ala Asn Asn Thr Glu Tyr Gly Tyr Gln Ile His Asp
           515                 520                 525
   Ser Leu Ser Gly Ser Met His Asp His Val Leu Asn Phe Lys Val Asp
       530                 535                 540
   Phe Asp Ile Ala Gly Val Glu Asn Thr Leu Val Lys His Ile Val Glu
   545                 550                 555                 560
   Pro Lys Glu Ile Lys Tyr Lys Trp Asn Asn Leu Thr Arg Ser Thr Met
                   565                 570                 575
   His Leu Val Arg Lys Glu Ile Thr Asn Glu Asp Glu Gly Lys Met Asn
               580                 585                 590
   Trp Ser His Asn Gly Gln Glu Gln Val Val Ile Val Asn Lys Asp Ala
           595                 600                 605
   Pro Asn Lys Tyr Gly Glu Pro Lys Gly Tyr Lys Ile Met Pro Ser Arg
       610                 615                 620
   Gly Gly Ser Gly Met His Leu Thr Ile Thr Asn Ser Ser Asn Leu Phe
   625                 630                 635                 640
   Asn Ser Gln Gly Phe Ala Thr His Gln Tyr Tyr Val Leu Lys Arg Lys
                   645                 650                 655
   Asp Ser Glu Leu Arg Ala Ser Asn Ala Trp Asn Asp Tyr Asp Thr Gln
   660 665 670
   His Pro Met Ile Asp Phe Ser Lys Tyr Phe Asp Gly Glu Asp Ile Glu
   675 680 685
   Gln Glu Asp Ile Val Met Tyr Phe Asn Leu Gly Met His His Val Pro
   690 695 700
   His Thr Gly Asp Leu Pro Asn Thr Val Phe Ser Thr Ala Gln Ser Gly
   705 710 715 720
   Met Met Ile Leu Pro His Asn Tyr Leu Leu Ser Asp Pro Ser Arg Gln
   725 730 735
   Ala Thr Gln Gln Ile Arg Ile Asp Tyr Asn Val Asn Ser Thr Thr Asp
   740 745 750
   Val Tyr Ser Trp Ala Ser Gln Ala Ala Thr Gly Glu Leu Asp Phe Ser
   755 760 765
   Gln Ile Thr Trp Asp Pro Tyr Thr Tyr Asp Gly Asp Val Ser Val Arg
   770 775 780
   Lys Phe Pro Tyr Asp Pro Gln Asn Pro Phe Asn Asn Thr Glu Ser Ile
   785 790 795 800
   Val
<210> 3
   <211> 797
   <212> PRT
   <213> Fusarium graminearum GLO1
<400> 3
   Met Gly Lys Glu Lys Thr Glu Leu Thr Met Arg Leu Ile Val Cys Leu
   1 5 10 15
   Gln Ala Ala Ser Leu Ile Phe Asp Arg Arg Val Leu Ala Arg Ser Thr
   20 25 30
   Ser Asp Gly Pro Ala Ala Arg Tyr Asn Ser Leu Asn Arg His Lys Val
   35 40 45
   Glu Thr Ser Gln Cys Asp Ser Asn Lys Glu Val Gly Val Lys Ala Pro
       50                  55                  60
   Trp Lys Asn Val Phe Gln Ser Leu Thr Asp Gln Glu Tyr Ala Asp Val
   65                  70                  75                  80
   Thr Ala Tyr Leu His Lys Gln Glu Glu Leu Asn Leu Thr Ala Ile Val
                   85                  90                  95
   Asn Ser Thr Ser Trp Asp Asn Val Ile Val Ser Met Asp Leu Leu Gln
               100                 105                 110
   Pro Asn Lys Thr Asp Ala Leu Thr Tyr Leu Glu Gly Asn Gly Pro Ala
           115                 120                 125
   Pro Ala Arg Tyr Ala Arg Ala Thr Leu Gln Phe Asn Ser Gln Leu Gln
       130                 135                 140
   Pro Tyr Ile Gln Glu Tyr Met Val Gly Pro Leu Pro Val Gln Glu Gly
   145                 150                 155                 160
   Ser Thr Arg Tyr Glu Glu Leu Asn Tyr Met Phe Ser Ser Gly Arg Gly
                   165                 170                 175
   Arg Ile Asn Val Tyr Asn Ala Asp Thr Glu Ala Ile Ala Glu Phe Asn
               180                 185                 190
   Leu Ala Val Gly Thr Glu Ile Gln Asn Ile Thr Lys Gln Leu Leu Asn
           195                 200                 205
   Gly Thr Ala Thr Gly Ala Lys Asp Asp Ser Leu Leu Ile Ala Gly Ser
       210                 215                 220
   Asp Pro Leu Ile His Asn Asp Asp Arg Val Tyr Gln Trp Asn Glu Phe
   225                 230                 235                 240
   Tyr Thr Ala His Thr Gly Gln Phe Phe Ser Glu Thr Ile Leu Pro Thr
                   245                 250                 255
   Ser Leu Gln Phe Lys Val Asp Ile Thr Gly Arg Asp Pro Ser Lys Trp
               260                 265                 270
   Lys Val Val Gly Trp Tyr Tyr Asp Gly Ser Tyr Trp Ser Thr Thr Ala
           275                 280                 285
   Glu Phe Lys Glu Gly Ser Lys Thr Leu Lys Arg Lys Pro Gly Pro Asn
       290                 295                 300
   Val Asp Gly Leu Trp Thr Ser Thr Asp Gln Gln Gly Asp Lys Leu Pro
   305                 310                 315                 320
   Leu Asp His Leu Gln Pro Pro Thr Ala Val Gln Pro Asp Gly Pro Arg
                   325                 330                 335
   Phe Arg Val Asp Arg Glu Glu Asn Tyr Ile Glu Trp Met Asp Phe Ser
               340                 345                 350
   Phe Phe Ile Ser Asn His Lys Glu Thr Gly Leu Gln Leu His Asp Val
           355                 360                 365
   Arg Tyr Arg Gly Glu Arg Ile Ile Tyr Glu Leu Gly Leu Gln Glu Ala
       370                 375                 380
   Met Ala His Tyr Ala Ser Gln Asp Pro Leu His Ala Ser Ser Ala Tyr
   385                 390                 395                 400
   Leu Asp Thr Ser Tyr Gly Ile Gly Thr Ser Gln Trp Asn Leu Val Asp
                   405                 410                 415
   Gly Phe Asp Cys Pro Ser His Ser Thr Tyr Leu Asn Thr Ser Phe Tyr
               420                 425                 430
   Ile Ser Glu Met Thr His Ile His Pro Asn Ser Leu Cys Leu Phe Glu
           435                 440                 445
   His Asp Thr Gly Tyr Pro Ile Gln Arg His Leu Thr Gly Thr Tyr Val
       450                 455                 460
   Ser Ala Thr Lys Asn Ile Val Phe Thr Val Arg Ser Val Ser Thr Val
   465                 470                 475                 480
   Gly Asn Tyr Asp Tyr Leu Phe Glu Tyr Thr Phe His Tyr Asp Gly Ser
                   485                 490                 495
   Ile Ser Val Thr Val Arg Ala Ser Gly Tyr Ile Gln Gly Ala Phe Trp
   500 505 510
   Ser Gly Asp Gly Asp Tyr Gly Phe His Ile His Asp Asn Leu Ser Gly
   515 520 525
   Ser Met His Asp His Val Ile Asn Phe Lys Leu Asp Leu Asp Ile Lys
   530 535 540
   Gly Arg Lys Asn Ser Val Leu Lys Thr Glu Phe Val Pro Val Ser Val
   545 550 555 560
   Val Tyr Pro Trp Ser Glu Gly Gln Ser Ile Asn Thr Met Lys Ala Asn
   565 570 575
   Arg Ser Tyr Ile Ala Ser Glu Asp Glu Gly Lys Ile Thr Trp Ala Lys
   580 585 590
   Asn Gly Ala Ala Ala Tyr Ala Val Val Asn Lys Asp Ala Leu Asn Asp
   595 600 605
   Phe Gly Glu Ala Pro Gly Tyr Ala Ile Ser Pro Asn Ser Gly Ser Thr
   610 615 620
   Gly His Leu Thr Val Gln Ser Ser Thr Ala Leu Gly Gln Ser Ala Asn
   625 630 635 640
   Trp Ala Asn His Asn Ile Phe Ala Leu Gln Gln His Asp Thr Glu Pro
   645 650 655
   Lys Ser Ala Tyr Ala Phe Asn Ser Tyr Asp Pro His His Pro Ala Val
   660 665 670
   Asp Phe Asn Lys Phe Phe Asn Gly Glu Ser Leu Asp Gln Glu Asp Ile
   675 680 685
   Val Leu Tyr Phe Asn Leu Gly Met His His Leu Pro Asn Thr Ala Asp
   690 695 700
   Leu Pro Asn Thr Val Thr Thr Lys Ala Val Ser Ser Met Met Ile Ser
   705 710 715 720
   Pro Gln Asn Tyr Phe Ser Gly Asp Ile Ser Arg Arg Thr Met His Gln
   725 730 735
   Val Arg Val Ser Phe Asp Asp Lys Ser Asn Val Thr Ala Val Asn Met
   740 745 750
   Phe Gly Thr Lys Gln Pro Thr Cys Ala Phe Asp Met Ala Lys Ala Ala
   755 760 765
   Pro Lys Leu Asp Thr Phe Val Gly Glu Leu Gln Ile Pro Lys Phe Pro
   770 775 780
   Phe Asn Pro Ser Gly Ser Leu Gln Thr Asn Pro Gly Gly
   785 790 795
<210> 4
   <211> 821
   <212> PRT
   <213> Podospora anserina ZGY
<400> 4
   Met Gly Phe Phe Asp Ser Leu Ser Pro Arg Ala Val Ala Thr Ser Ala
   1 5 10 15
   Leu Leu Leu Thr Ser Phe Ala Gln Asp Ala Leu Ala Arg Pro Asn Pro
   20 25 30
   Asp Pro Lys Ala Pro Trp Val Lys Asn Thr Gly Leu Gly Lys Lys Lys
   35 40 45
   Met Ala Asn His Leu Lys Arg Ala Ile Met Gly Asp Arg Arg Ala Val
   50 55 60
   Glu Thr Pro Phe Cys Ser Glu Thr Leu Ala Thr Glu Ile Lys Ala Pro
   65 70 75 80
   Lys Pro Asn Val Trp Gly Pro Leu Val Asp Val Glu Val Ala Ser Val
   85 90 95
   Val Glu Trp Leu Phe Ala Gln Ala Asp Leu Asn Leu Thr Val Thr Glu
   100 105 110
   Glu Ala Gly Gly Trp Asp Asn Thr Ile Gln Leu Val Glu Ala Met Trp
   115 120 125
   Pro Asn Lys Thr Asp Val Leu Ala Phe Val Asp Gly Asp Gly Pro Ala
       130                 135                 140
   Pro Thr Lys Tyr Ala His Val Val Leu Asn Asn Arg Ala Thr Glu Thr
   145                 150                 155                 160
   Pro His Tyr Ala Asp Ile Ile Val Gly Pro Leu Pro Leu Asp Asn Ala
                   165                 170                 175
   Thr Ala Lys Trp Glu Pro Leu Glu Tyr Pro Tyr Thr Lys Gln Asn Gly
               180                 185                 190
   Gly Lys Val Arg Asn Leu Asp Ala Asp Ser Asp Arg Leu Tyr Ser Glu
           195                 200                 205
   Trp Leu Phe Lys Ile Gly Ala Ser Val Ala Asp Ile Thr Leu Asp Leu
       210                 215                 220
   Trp Asn Gly Thr Ala Met Gly Leu Glu Asn Asp Thr Leu Ser Ile Trp
   225                 230                 235                 240
   Gly Ile Asp Pro Leu Trp Gln Asp Asp Gly Glu Ile Ile Arg Trp Asp
                   245                 250                 255
   Thr Phe Trp Asn Ile Pro Thr Gly Asp Phe Asp Asp Met Thr Leu Phe
               260                 265                 270
   Pro Leu Gly Leu Tyr Phe Ser Ser Glu Val Ser Gly Arg Asp Pro Ser
           275                 280                 285
   Lys Trp Glu Leu Arg Gly Trp Leu Tyr Asn Ser Val Phe Tyr Ala Thr
       290                 295                 300
   Thr Glu Glu Phe Arg Ala Ala Tyr Trp Ser Glu Asp Phe Val Lys Asn
   305                 310                 315                 320
   Gly Pro Asn Val Asp Gly Asp Trp Ala Arg Thr Asp Lys Asn Gly Glu
                   325                 330                 335
   Thr Pro Glu Met Asp Lys Ala Gln Gly Pro Val Ile Val Ala Pro Ala
               340                 345                 350
   Gly Ala Arg Phe Ala Val Asp His Lys Glu Lys Tyr Val Glu Trp Met
           355                 360                 365
   Asp Trp Ser Phe Tyr Val Gly Phe Asn Arg Asp Thr Gly Pro Gly Leu
       370                 375                 380
   Phe Asp Ile Arg Tyr Lys Gly Glu Arg Leu Val Tyr Glu Leu Ser Leu
   385                 390                 395                 400
   Gln Glu Ala Leu Ala His Tyr Ala Gly Asn Asp Pro Met Asn Ser Gly
                   405                 410                 415
   Thr Ala Tyr Leu Asp Ser Phe Tyr Gly Phe Gly Pro Tyr Thr Phe Glu
               420                 425                 430
   Leu Val Lys Gly Tyr Asp Cys Pro Ala Tyr Ala Thr Tyr Met Asn Thr
           435                 440                 445
   Ser Phe Tyr Val Asn Glu Glu Thr Arg Thr His Ile Asp Ser Leu Cys
       450                 455                 460
   Leu Phe Glu Tyr Val Ala Asp Tyr Pro Met Gln Arg His Thr Thr Ser
   465                 470                 475                 480
   Asp Tyr Val Ser Val Thr Lys Asn Thr Tyr Phe Val Ile Arg Ser Ile
                   485                 490                 495
   Ala Thr Ile Gly Asn Tyr Asp Tyr Gln Gln Ser Phe Ser Phe Phe Met
               500                 505                 510
   Asp Gly Ser Phe Ala Val Glu Val Arg Ala Ser Gly Tyr Ile Gln Ser
           515                 520                 525
   Ala Tyr Phe Ala Gly Asn Glu Asp Tyr Gly Phe Lys Ile His Asp Asn
       530                 535                 540
   Leu Ser Gly Ser Met His Asp His Val Leu Asn Phe Lys Ala Asp Phe
   545                 550                 555                 560
   Asp Val Leu Gly Thr Asn Asn Ser Ile Glu Leu Met Ser Met Val Pro
                   565                 570                 575
   Val Ser Arg Ser Tyr Pro Trp Ser Ala Gly Lys Val Arg Asn Thr Met
               580                 585                 590
   Ala Leu Glu Arg Lys Phe Ile Glu Thr Glu Asp Glu Ser Arg Phe Asn
           595                 600                 605
   Trp Gly Pro Asn Ser Ala Thr Gln Val Leu Ile Val Asn Glu Asn Glu
       610                 615                 620
   Arg Asn Lys His Gly Glu Met Arg Gly Tyr Arg Val Leu Pro Tyr Met
   625                 630                 635                 640
   Gly Thr Ala His Leu Thr Val Gln Asn Ser Thr Asn Leu Gly Val Ala
                   645                 650                 655
   Ala Gln Trp Ala Asn His Asp Val Gln Ile Thr Lys Tyr Lys Asp Ser
               660                 665                 670
   Glu Gln Lys Ala Tyr His Ala Phe Asn Thr Gln Asp Val His Asp Pro
           675                 680                 685
   Pro Val Asn Phe Asp Lys Tyr Phe Asp Gly Glu Ser Val Arg Asn Glu
       690                 695                 700
   Asp Ile Val Leu Trp Leu Asn Leu Gly Met His His Val Pro His Thr
   705                 710                 715                 720
   Gly Asp Leu Pro Asn Thr Val Gln Thr Thr Ala His Ser Gly Ile Gln
                   725                 730                 735
   Phe Met Pro Ser Asn Tyr Phe Asp Ile Asp Gln Ser Arg Arg Thr Val
               740                 745                 750
   Asn Gln Val Arg Ile Asn Tyr Phe Asn Gly Ser Ala Glu Val Glu Glu
           755                 760                 765
   Phe Gly Gln Phe Lys Val Gly Ser Ser Glu Gly Thr Cys Ser Thr Cys
       770                 775                 780
   Lys Leu Asn Tyr Thr Pro Leu Glu Pro Glu His Gln Gly Tyr Lys Gly
   785                 790                 795                 800
   Asp Val Val Ile Arg Lys Phe Pro Phe Asp Pro Asn Asn Pro Phe Tyr
                   805                 810                 815
   Ala Thr Ser Gly Ile
               820
<210> 5
   <211> 770
   <212> PRT
   <213> Fusarium graminearum GL02
<400> 5
   Met Arg Pro Ser Gly Leu Leu Val Ser Leu Ser Leu Val Pro Leu Ala
   1               5                   10                  15
   Leu Ala Arg Ala Phe Pro Pro Ala Arg Leu Glu Ser Arg Lys Glu Asp
               20                  25                  30
   Ser Cys Ser Ser Val Gln Pro Ser Ala Ser Ala Pro His Lys Asn Phe
           35                  40                  45
   Trp Gly Ser Leu Thr Lys Lys Glu Thr Ala Asp Val Leu Ala Phe Leu
       50                  55                  60
   His Arg Asp Thr Thr Gly Phe Asn Leu Thr Val Ala Glu Asn Ala Thr
   65                  70                  75                  80
   Ser Arg Asp Asn Lys Ile Met Ser Val Glu Leu Met Met Pro Asn Lys
                   85                  90                  95
   Ser Asp Thr Leu Pro Phe Leu Ser Asp Lys Ala Gly Ser Pro Thr Arg
               100                 105                 110
   Tyr Ala Leu Ala Ala Val Met Phe Gly Val Pro Glu Lys Ala Tyr Leu
           115                 120                 125
   Gln Glu Phe Lys Val Gly Pro Leu Pro Ile Thr Asn Ala Ser Ser Val
       130                 135                 140
   Thr Pro Phe Thr Phe Ala Asn Thr Lys Lys Gly Asp Gly Lys Ile Ala
   145                 150                 155                 160
   Val Val Asn Pro Asp Ala Glu Asp Tyr Gly Asn Phe Asn Leu Lys Ile
                   165                 170                 175
   Met Lys Glu Ala Glu Asp Val Thr Lys Leu Leu Trp Asn Leu Thr Val
               180                 185                 190
   Asp Asp Gly Leu Gln Ile Pro Leu Ala Phe Ala Ala Pro Ile Asn Val
           195                 200                 205
   Thr Asp Gly Lys Val Ile Met Trp Gln Gly Phe Asn Ala Pro Val Thr
       210                 215                 220
   Ser Ile Tyr Asp Thr Ile Ser Leu Leu Pro Leu Gly Leu Tyr Leu Arg
   225                 230                 235                 240
   Thr Asp Ile Thr Gly Arg Asp Pro Ser Gln Trp Lys Val Thr Gly Trp
                   245                 250                 255
   Val Tyr Gly Asn Glu Phe Tyr Lys Asp Leu Asp Gly Leu Arg Ala Ala
               260                 265                 270
   Val Ala Lys Pro Asp Phe Lys Pro Leu Gly Met Asn Leu Asp Gln Pro
           275                 280                 285
   Trp Ala His Thr Asn Lys His Gly Asp Asp Leu Pro Leu Asp Asp Glu
       290                 295                 300
   Ala Pro Pro Ala Asn Val Gln Ser Gly Lys Ser Arg Phe Ala Val Asp
   305                 310                 315                 320
   Glu Asp Glu Ser Tyr Val Thr Trp Met Asp Phe Ser Phe Tyr Thr Ser
                   325                 330                 335
   Ile Thr Arg Asp Asn Gly Leu Arg Leu Tyr Asp Val Lys Tyr Lys Gly
               340                 345                 350
   Lys Arg Ile Leu Tyr Glu Leu Gly Leu Asp Glu Ala Ile Ala His Tyr
           355                 360                 365
   Ala Gly Ile Asp Pro Val Gln Ser Gly Ile Cys Tyr Phe Asp Ser Met
       370                 375                 380
   Ser Gly Phe Gly Pro Ala Met Ile Ser Leu Val Lys Gly Tyr Asp Cys
   385                 390                 395                 400
   Pro Ala Tyr Ser His Tyr Ser Asn Val Thr Gln Thr Thr Gly Glu Thr
                   405                 410                 415
   Thr Phe Thr Gln Lys Asp Ala Leu Cys Met Phe Glu Ile Asp Lys Gly
               420                 425                 430
   Phe Pro Ile Gln Arg His Ser Trp Ala Gly His Thr Thr Val Thr Lys
           435                 440                 445
   Asn Ile Ala Phe Asn Ile Arg Ala Ile Tyr Thr Ile Gly Asn Tyr Asp
       450                 455                 460
   Tyr Met Met Thr Tyr Gln Phe His Leu Asp Gly Ser Ile Glu Ile Asp
   465                 470                 475                 480
   Val Arg Ala Ser Gly Tyr Ile Ser Ser Ala Phe Tyr Ala Glu Asn Glu
                   485                 490                 495
   Asp Tyr Gly Phe Lys Ile His Asp Ser Leu Ser Gly Ser Leu His Asp
               500                 505                 510
   His Val Ile Thr Phe Lys Ala Asp Phe Asp Ile Leu Gly Glu Lys Asn
           515                 520                 525
   Ser Leu Gln Lys Ile Asp Ile Lys Pro Ser Thr Glu Lys Tyr Lys Trp
       530                 535                 540
   Ser Asp Gln Thr Arg Asn Thr Met Lys Ala Val Lys Ser Phe Ile Asp
   545                 550                 555                 560
   Asn Glu Asp Asp Ala Lys Ile Asn Trp Ser Pro Asn Gly Ala Thr Met
                   565                 570                 575
   Tyr Ala Val Val Asn Lys Asp Glu Lys Asn Pro Tyr Gly Glu Ser Pro
               580                 585                 590
   Gly Tyr Arg Val Ala Pro Ala Ser Gly Val Ala Tyr Leu Thr Val Gln
           595                 600                 605
   Asp Ser Ser Val Met Gln Asn Ala Gly His His Thr Thr His His Leu
       610                 615                 620
   Tyr Ala Thr Arg Gln Lys Asp Asn Glu Leu Tyr Ala Val Gly Ala Tyr
   625                 630                 635                 640
   Asn Ser Leu Thr Pro Glu Asp Pro Gln Val Asp Phe Asn Glu Tyr Phe
   645 650 655
   Asn Ser Glu Ser Leu Asp Gln Glu Asp Ile Val Leu Trp Phe Asn Leu
   660 665 670
   Gly Met His His Met Pro His Thr Gly Asp Leu Pro Asn Thr Val Phe
   675 680 685
   Ser Thr Ala His Ser Ala Met Leu Ile Glu Pro Phe Asn Tyr Leu Met
   690 695 700
   Gly Asp Pro Ser Gln Ala Ser Ser Gln Gln Val Leu Ile Lys Thr Lys
   705 710 715 720
   Lys Asp Gly Lys Pro Glu Ile Val Thr Tyr Gly Ala Lys Asn Ala Thr
   725 730 735
   Cys Ala Ile Asp Met Ala Gln Leu Asn Pro Asp Leu Ser Asn Tyr Ser
   740 745 750
   Asn Ser Ile Ser Val Leu Lys Tyr Pro Phe Asp Gly Ser Lys Pro Asp
   755 760 765
   Arg Leu
   770
<210> 6
   <211> 2481
   <212> DNA
   <213> Aspergillus nidulans ZGR synthetic DNA
<400> 6
   ttaattaaca ccgtcaaaat gaagctccct ctgtcgttct ccggtggtat tgcgttgctc 60
   ctgattggat cgcttgctgg agatgtggtg gccaccaagt tggatcctag ccgtgatctc 120
   gttcgccaga agtcccgcgg ccgtaagaac cagatgcgct cgctggtcgg ccgtagcaac 180
   cagcacatca ctcaccagtc tcgcccctac accaacgaat acgcttctcc ctgccagatc 240
   acccctcccc aggagattaa ggcgcctaag gaaaacgtct ggtacggtct cactgacgac 300
   gaaaccgctg acgtggccaa gtggcttttc ggccgtcccg agcttaacct caccaccacc 360
   gagaacgctg gcgaatggga taacaccatc gctcttatcg agctgcaccg ccctaacaag 420
   agcgaggcca tcccttactt ggatggcagc ggtcccgcgc ccacccgtca cgcccacgtt    480
   cgtctcaaca accgcgccac caccgatccc tacttcgccg acattctcgt gggtcccctg    540
   cccgtctcca acgctactac ttgggagcct ctggaattcc cttacactcg taagactcag    600
   ggtcaggtcc gcaacgtcga acccgacgga gagaccgtct actcggagtg gcttttcaag    660
   atctctgcct ctatcgcgga catcaccctg gacctttgga acggtaccgc ccttggtctg    720
   gagaacgaca ctctcgacat ctggggaatc gatcccttgt ggcaggatga cggacgcatc    780
   attcgctggg acatgttctg gaacatggcc gatgacgagt tcgactcgga gaccttgctg    840
   cctctcggcc tctacctcaa gtccgacgtc actggccgcg atcctagcca gtggaagttg    900
   ctgggttgga tgtacaacga cattttctac gagactactg aggaattccg taaggcctac    960
   tggtcccccg gtttcgttaa gctgaagccc aacgttgacg gcgcttgggc ccacaccgag   1020
   cagcgcggtc ccgtgccccc ccaggaccgt aagcagcccc ccgtcatgat cgctcctgac   1080
   ggcgctcgtt actctgtcga cgccgagcgt aagtacgtta cctggatgga tttctccttc   1140
   tacatcgctt tcaaccgcga taccggtctg tccctcttcg acatcaagta caagggtcag   1200
   cgtgtgctct acgaactcgg tcttcaggag gctctggccc actacgctgc caacgatccc   1260
   gtccagtcct ccgttgcgta cctcgattcc tactacggtt tcggccccta cgccttcgag   1320
   cttctcaagg gctacgactg cccctcgtac gcgtcctacc tcaacacttc cttctacaag   1380
   gacgaagaga cccacaccca cgttgactct ctctgcctgt tcgagttcga tgccgattac   1440
   cctatggccc gccacagcac ctccgagttc gtcagcgtca ccaagaacgt ctacttcacc   1500
   ctgcgtagcg tttcgaccat cggaaactac gactacatgt tctcctacaa cttccacatg   1560
   gacggtacta ttggcgtcga ggtccgcgcc tctggctaca tccagagcgc ctactacgcc   1620
   aacaaccagg acttcggcta ccagatccac gattccctgt ctggttccat gcacgaccac   1680
   gtcttgaact tcaaggctga cttcgacatc cttggtccta acaacactat tgagctcgtc   1740
   tccgtcgttc ccgtcaccaa gcagttctcc tggtcgggta acaagacccg caacaccatg   1800
   cagctgggac gttctttcat ccactccgag gatgaggccc gcctcaactg gggtttcaac   1860
   ggccagaccc agctccacgt cgtgaaccag gacaagccca acaagttcgg cgagcctcgt   1920
   ggttaccgca tcctcccctc cgccggtacc gctcacttga ccgttctgaa ctccagcaac   1980
   cttgttcacg ctgcccactg ggcggagtac gacgtgcagg ttacccgcca gcacgatttc   2040
   gagcctactt ccgctcaccc ctacaactct caggatatcc acaacccccc cgtcgacttc   2100
   tccaccttct tcaacggtga atccctgaac cagactgacc tcgtcgtctg gctgaacctc   2160
   ggtatgcacc acgttcctca caccggagac ttgcccaaca ctgtcttcac caccgctcac 2220
   agcggcgtcg ccttcacccc tcttaactac ctccccggtg acccctctcg tgagaccgtc 2280
   aacatggtgc gtgtcgacta ctcggatggc gccgccaccg ccgttcgcac cttcggccag 2340
   tccaacgaaa cttgcagcgt tgtgctgcag cccgtcgaga acgagctctg gtcttaccag 2400
   ggtgatgtcg tcgtccgtaa gttcccttac gaccccaacg atcccttcta cgagaccgac 2460
   tccgacgctt aaaggcgcgc c 2481
<210> 7
   <211> 2433
   <212> DNA
   <213> Cryptococcus neoformans ZGT synthetic DNA
<400> 7
   ttaattaaca ccgtcaaaat gcgcgctatt gccctccttt acttgctggg caccgccttc 60
   gctgttccta gccctaagat ttcgaacgtg aagaagcccg gccactacca ccacccccac 120
   cgtcgtgaca tcgtcactaa cgaggtcgat gcgtccgctt cctctcccac cacttcggcc 180
   cccaaggata acatctggaa cttcctctcc aacgatgaag cggctggaat tatcgcgttc 240
   ctccactctc agaccgagct gaacctcact gccgtgaacg acgctggaga ttgggacaac 300
   accattaccg ttgtcgacct gcttccccct aacaagaccg aagccctttc ttacatggac 360
   ggcaacggaa ctaagcccga gcgctggggc atcgcctctc tcctttgcgg tgccaccgag 420
   gagccctacg ctcaggattt ggtggtggga cctctgcctg ttagcgaggt taccatctac 480
   tacccctaca cttacggtac ccacgctccc gacgccaaga tccgcgtgta cgacatggac 540
   gataactcgg aattccttag cgatatcgcc atgagcatga aggacatcat ctccgatatc 600
   ctgaacgcta ccattgacac tgccgacgac ctcgccgaca ccttcgacat ttggggtatt 660
   gaccccctct ggcaccagcc tgatgagaac ggtaacgatc gtgtcatcta ctgggctggt 720
   ttctggcgtt accctgattc cctgcagatg gagaactcca ctatcaactt cgacggcgag 780
   accctcctcc cccagggttt gtacatccag actgacatca ccggtcgcga ccgttccaag 840
   tgggcgctca tgggtatcct gtacggtgat gactactaca cctccgtgga cgaattccgc 900
   gctgcctggc gtaagcccgg tttcaagaag ttcactccca actacagcgg cgattggatc 960
   ggcaccgacc agaccggtga tgtcatgccc ttcgagaccg aagcccctcc tatgaacgtc 1020
   cagcccggcg gccagcgctt caaggtcgac gaagacaaca agtacgttga gtggatggac 1080
   ttcagcttct acctgacctt cacccgcgac accggaatgc gtctttacga cgtcaagttc 1140
   aagggtgagc gtatcatcta cgagttggga cttcaggagg ccatcgctca ctacgccggt   1200
   aacgaccccg tccagtctgg cactgcgtac ctggatacct actacggctt cggcccctac   1260
   gctttctccc aggtccctgg tttcgacatg cccctctacg cctactgcat gaacacttcg   1320
   ttccacgctg ccgagctctc tacctcccac cgctgcggca tctccatttt cgaggccgat   1380
   cagaactacc ccatccagcg ccactcgaac atgaactacg tctctgccac taagaacatc   1440
   gctctgactc tgcgctccat ctccaccgtt ggtaactacg attacaactt cgactacaac   1500
   ttctacctcg acggtaccat tgagaccgtc gtccgtgcct cgggttacat tcagtccgct   1560
   ttctacgcta acaacaccga gtacggctac cagatccacg actccctcag cggttccatg   1620
   cacgatcacg ttttgaactt caaggttgat ttcgatatcg ccggtgtcga gaacaccctg   1680
   gtcaagcaca tcgttgaacc taaggaaatc aagtacaagt ggaacaacct cacccgtagc   1740
   accatgcacc tggtgcgcaa ggagatcacc aacgaggacg agggcaagat gaactggtcc   1800
   cacaacggac aggagcaggt cgtgatcgtc aacaaggacg cccctaacaa gtacggtgag   1860
   cccaagggct acaagattat gccctctcgc ggtggttctg gtatgcactt gaccatcact   1920
   aacagctcca acctcttcaa ctcgcagggc ttcgctaccc accagtacta cgtccttaag   1980
   cgtaaggact ctgaactgcg tgcgtccaac gcttggaacg actacgatac tcagcacccc   2040
   atgattgact tcagcaagta cttcgacggt gaggatatcg agcaggaaga catcgttatg   2100
   tacttcaacc tcggcatgca ccacgttccc cacaccggtg acctccccaa caccgtgttc   2160
   tccaccgccc agtccggaat gatgatcctt cctcacaact acctgctgag cgacccctct   2220
   cgccaggcca ctcagcagat ccgtattgat tacaacgtca actccaccac cgacgtttac   2280
   tcctgggcct cgcaggctgc cactggcgag ctcgatttct ctcagatcac ctgggaccct   2340
   tacacctacg atggtgacgt cagcgttcgc aagttcccct acgaccctca gaaccccttc   2400
   aacaacaccg agtccatcgt ctaaaggcgc gcc                                 2433
<210> 8
   <211> 2762
   <212> DNA
   <213> Fusarium graminearum GLO1 genomic DNA
<400> 8
   ttaattaact cataggcatc atgcgattaa tcgtctgctt acaagctgct tcgcttatat     60
   ttgataggag agttttggct cgttctacct ccgatggtcc agcagcaagg tacaattcct    120
   tgaaccgtca caaagtcgaa acgtcacaat gtgattccaa caaggaagtt ggtgtcaagg    180
   ccccatggaa aaatgttttc cagagcctga cagatcaaga gtatgctgat gttactgcat    240
   atcttcacaa gcaggaagaa ctcaacctga ccgctattgt gaactcgaca tcgtgagtct    300
   tgaacttttt actttctcca tcctactgat tttttcgaaa atcctttgct ctgctgtcta    360
   cctttgcaca tcaacaagtc ttggctgact ttataaagtt gggataatgt catcgtgtct    420
   atggacttgt tgcagccaaa caagactgat gccttgacat atctcgaagg taatgggcca    480
   gctccagctc gatatgcgcg ggccacgctg caattcaact cgcaactgca accatacatt    540
   caagagtaca tggttggccc attacctgtt caggaagggt ccactcgata cgaagagctc    600
   aattacatgt tctcatctgg acgtggtcgc attaacgtgt acaacgctga tactgaggcc    660
   attgctgagt tcaaccttgc tgttggaacg gaaatacaga acataaccaa gcagcttctc    720
   aatggcgtaa gtaaccgatg tccggattcg ttttcgatgc tccatgctaa tgacgccaac    780
   agactgctac aggtgctaaa gacgacagtc ttctaatcgc aggcagtgat cctctgatcc    840
   acaacgatga cagggtctat cagtggaacg agttctacac tgctcacacg ggtcaattct    900
   tttcagagac gatcctccct accagtctgc agttcaaagt cgatatcacc ggtcgagatc    960
   cttccaaatg gaaggtggtc ggttggtatt acgacggcag ctattggtcg acgaccgccg   1020
   agtttaaaga gggatctaag acgctcaaga gaaagccagg gcccaacgtt gatgggcttt   1080
   ggacctccac tgaccagcaa ggtgacaagt taccgcttga ccacctgcaa ccccctactg   1140
   ctgttcaacc cgatggtcca cgatttcgtg tggatagaga ggagaattac attgagtgga   1200
   gtaagtgcac attgtaatgt tttgaatcac gctttagttg acaaccgtat agtggacttt   1260
   agctttttca tctcaaacca caaagagact ggtctgcaac tccatgatgt cagatataga   1320
   ggcgagcgta tcatctatga gcttggcctc caggaagcga tggctcatta cgcttcccag   1380
   gatccgctgc atgcctcgtc agcatatctg gacacttcat acggcattgg caccagccag   1440
   tggaaccttg tcgacggctt tgattgtcct tctcactcca cctacctcaa cacatctttt   1500
   tacatcagcg agatgactca catccacccc aacagcttgt gtcttttcga acatgatact   1560
   ggatatccta tacagcgaca tttgacaggg acgtatgttt ccgcgaccaa gaacattgta   1620
   tttactgttc gcagcgtctc tacagttggc aactatgact atttgttcga gtataccttt   1680
   cattacgacg ggtccattag cgtcactgtt agagcgtctg gctatatcca gggagcgttt   1740
   tggtccggag atggcgatta tggctttcat atccatgata acctgtcagg ttctatgcat   1800
   gaccatgtca ttaactttaa gctcgatctg gatatcaaag gcaggaagaa tagtgtgtta   1860
   aagactgagt ttgtgcccgt ctcagtagtg tacgttgttt tccatccatt atactaccat 1920
   gtactaactt gaacagatac ccctggtctg agggacaatc aatcaatact atgaaggcca 1980
   accggtcgta tatcgccagt gaggacgagg gcaagattac atgggcgaag aatggagcag 2040
   ctgcatatgc agttgtcaac aaggatgctc tgaatgattt cggagaggca cctggctacg 2100
   cgatttctcc aagtaagtat cccctacatg gatacaaacc tggacgacac taaccggctt 2160
   cgcagacagc ggtagcacag ggcacttgac ggtgcagtca tctacagctc ttggacagtc 2220
   cgcaaactgg gccaatcaca acattttcgc cctgcagcaa cacgacaccg agccgaaaag 2280
   cgcttacgct ttcaatagtt atgatccgca ccacccagct gtagacttca ataaattctt 2340
   caatggggag agtctcgatc aagaagacat cgttttgtaa gtaaccctta ctgagcatta 2400
   ctattacccg agactaacag ttacgcaaga tacttcaacc tcggcatgca ccacctacct 2460
   aatacggctg atctgcccaa cactgtgacg acgaaagccg tttcgtcgat gatgatttca 2520
   cctcagaatt acttttcagg cgatatctca cgacggacca tgcatcaagt gcgtgtctcc 2580
   ttcgatgaca agtcaaatgt cactgctgtc aacatgtttg gaacgaagca gcctacttgc 2640
   gcgtttgata tggccaaggc cgcacccaag ttggatacct tcgttgggga gcttcagatt 2700
   cctaaatttc ctttcaaccc aagtggaagt cttcagacaa accccggtgg ttaaggcgcg 2760
   cc 2762
<210> 9
   <211> 2493
   <212> DNA
   <213> Podospora anserina ZGY synthetic DNA
<400> 9
   ttaattaaca ccgtcaaaat gggcttcttc gactctctgt cccctcgcgc ggtggctact 60
   tcggctctcc ttttgacctc cttcgcccag gacgctctcg cgcgtcccaa ccccgatcct 120
   aaggccccct gggtcaagaa caccggtctg ggaaagaaga agatggccaa ccaccttaag 180
   cgcgccatca tgggtgaccg tcgcgctgtt gaaaccccct tctgcagcga aactttggcc 240
   accgagatca aggcccccaa gcctaacgtt tggggtcctc tcgtcgatgt cgaggtcgct 300
   tccgttgtcg aatggctctt cgcccaggct gatctgaacc tgaccgtgac tgaggaggct 360
   ggaggctggg ataacaccat ccagcttgtc gaggctatgt ggcctaacaa gaccgatgtc 420
   ctcgccttcg tggacggtga cggacccgcg cccactaagt acgctcacgt tgttctcaac 480
   aaccgtgcta ccgagactcc ccactacgcc gacattatcg tgggtcccct ccccttggac 540
   aacgccactg ccaagtggga gcctcttgag tacccctaca ctaagcagaa cggtggcaag 600
   gttcgcaacc tggacgctga ctccgatcgt ctgtacagcg agtggctgtt caagattgga 660
   gcctctgtcg ccgacatcac ccttgacctc tggaacggta ccgccatggg cctcgagaac 720
   gacaccctgt ccatttgggg catcgaccct ctctggcagg atgacggcga gattatccgt 780
   tgggatacct tctggaacat ccctaccggt gatttcgacg acatgaccct gttccccttg 840
   ggcctctact tctcgtccga agtctctggt cgtgacccct ccaagtggga gcttcgcggc 900
   tggctgtaca acagcgtctt ctacgcgacc accgaggagt tccgcgctgc ctactggagc 960
   gaagatttcg tgaagaacgg tcccaacgtc gatggtgact gggctcgcac tgacaagaac 1020
   ggtgagaccc ccgaaatgga caaggcccag ggccctgtta ttgtcgctcc tgccggagct 1080
   cgtttcgctg tcgatcacaa ggaaaagtac gttgaatgga tggattggtc gttctacgtc 1140
   ggtttcaacc gcgacaccgg tcccggcctc ttcgatatcc gttacaaggg tgagcgtttg 1200
   gtctacgagc tttctctcca ggaggccctg gcgcactacg ccggcaacga ccctatgaac 1260
   tccggtaccg cctacctgga cagcttctac ggtttcggcc cctacacttt cgagctcgtt 1320
   aagggttacg actgccccgc ctacgccacc tacatgaaca cctccttcta cgtgaacgag 1380
   gaaacccgca ctcacatcga ttccctctgc ctcttcgagt acgtcgctga ctaccccatg 1440
   cagcgccaca ccacctctga ttacgtgagc gtcaccaaga acacttactt cgtcatccgt 1500
   tcgatcgcta ccatcggcaa ctacgactac cagcagagct tctctttctt catggacggt 1560
   tccttcgcgg tcgaggttcg cgcctccgga tacattcagt ccgcttactt cgccggtaac 1620
   gaggactacg gcttcaagat ccacgataac cttagcggat cgatgcacga ccacgtcctg 1680
   aacttcaagg ccgacttcga cgtgttgggc accaacaact ccattgagct tatgtctatg 1740
   gtccccgtct cccgctccta cccctggtct gctggtaagg tccgtaacac catggccctg 1800
   gagcgcaagt tcatcgagac tgaagatgag tcgcgtttca actggggtcc taactccgct 1860
   acccaggttc tcatcgttaa cgagaacgag cgtaacaagc acggcgaaat gcgcggctac 1920
   cgtgtgctcc cttacatggg caccgcccac ctgactgtcc agaacagcac caacttgggt 1980
   gtcgctgcgc agtgggccaa ccacgacgtt cagatcacca agtacaagga ttccgagcag 2040
   aaggcttacc acgccttcaa cactcaggac gtccacgatc cccccgttaa cttcgacaag 2100
   tacttcgacg gagaatctgt ccgcaacgag gatattgtcc tctggctgaa ccttggtatg 2160
   caccacgtgc ctcacaccgg tgacctcccc aacactgttc agaccaccgc ccacagcggc 2220
   atccagttca tgccctccaa ctacttcgac atcgatcagt cgcgccgtac cgtcaaccag 2280
   gtgcgtatta actacttcaa cggttccgct gaggtcgagg aattcggaca gttcaaggtc   2340
   ggtagctctg agggcacctg ctccacttgc aagctgaact acacccctct cgagcccgag   2400
   caccagggtt acaagggcga cgttgtcatc cgcaagttcc ccttcgaccc taacaacccc   2460
   ttctacgcca cctccggtat ctaaaggcgc gcc                                 2493
<210> 10
   <211> 2965
   <212> DNA
   <213> Fusarium graminearum GL02 genomic DNA
<400> 10
   ttaattaact cataggcatc atgcgtccct caggtctcct ggtctcgctt tcgctggtgc     60
   ctctggctct agccagagct tttcctcccg caaggttgga aagtcgcaag gaggactctt    120
   gttcgtcggt ccagccgtct gcgagtgctc ctcacaagaa cttctggggt tctctgacca    180
   agaaagagac tgctgatgtt ctcgcatttt tgcatcgcga tacaacaggt ttcaatctga    240
   ccgttgcaga gaatgctact aggtatttat tggatcctat gtgattttga aaccactaac    300
   agttcattat agtcgcgata acaagatgtg agtcttgtac tatgaaccat gattttaacc    360
   tcaactcaca aggttttagc atgtccgttg agctcatgat gcccaacaag agtgatactc    420
   tcccattctt gtcagacaaa gcgggtagcc caacacgata cgccctagct gcggtcatgt    480
   ttggcgtacc tgaaaaggct tatctccagg aattcaaagt tggtccgttg cccatcacta    540
   atgcatcaag tgttacacct tttacttttg caaatacgaa gaagggcgac ggtaagatcg    600
   cagtcgtcaa ccctgacgca gaggattatg gcaacttcaa cctcaagatt atgaaggagg    660
   cagaggatgt gaccaagctt ctctggaatt tggtgagtta tgtgttttga attccatgtt    720
   tctttatcta acatattcaa gacggtcgat gatggactcc aaatcccgtt agcctttgca    780
   gcccctatca atgtcaccga tggaaaggtc atcatgtggc aaggcttcaa cgcgccagtt    840
   accagcatct acgacacaat ctcactgctt ccactgggtc tgtatttgcg aacagacatc    900
   accggccgcg atcctagtca atggaaagtc actggttggg tatacggtaa tgaattctac    960
   aaggatctcg atggtttgcg tgcagctgtc gccaagcccg atttcaaacc cctgggaatg   1020
   aacttggacc agccttgggc ccacacaaac aaacacggcg atgatctgcc cctggacgat   1080
   gaagctccac cagccaatgt tcaatccgga aagtcgagat ttgctgttga tgaagatgag   1140
   agctatgtga cttggagtga gtagcttctg attttgacta actgttacac tttactaatt   1200
   cgatacgtca gtggactttt ctttctacac tagcattact cgagacaatg gactacgact   1260
   gtatgatgtt aagtacaagg gcaagaggat tctctacgag gtatgcatga attgcaaaat 1320
   gactacgtct taaactaaca tttctagctt gggctggacg aggcgattgc acattatgcg 1380
   taagttgtga aagtaccctg gcctcacgcc actgttattg acaccatttg cagcggtatc 1440
   gatcctgtac aatccggaat ttgttatttc gacagcatgt ctggattcgg acccgcaatg 1500
   atctctctcg tgaaaggtaa gaattaaaaa tgactcgaga attgttttat cacattgcta 1560
   acacgataca ggttacgact gccccgccta ctcgcactac agcaatgtca ctcagactac 1620
   aggtgaaacg acgttcactc agaaggacgc tctttgtagg ttgagatctc atgtcaagca 1680
   attccacgct aatgaataat caaggcatgt ttgagatcga caagggcttc cccatccaac 1740
   gacactcttg ggccggccac accaccgtga caaagaacat tgccttcaac atccgtgcca 1800
   tctacacaat cggaaactac gactacatga tgacctacca gtttcacctc gacggttcta 1860
   tcgaaatcga tgtcagagca tcaggctaca tctcgtcggc cttctacgct gagaacgaag 1920
   actacggttt caagatccac gacagccttt ccggatctct tcatgatcat gtcatcacat 1980
   tcaaagccga ctttgacatc ttgggcgaga agaactcttt gcaaaagatt gatatcaagc 2040
   catcaactga gaagtgagta tccattttgt aacagggttt cttttacatc ttactaatct 2100
   ggaatagata caagtggtct gatcagacac gcaacactat gaaggcagtc aagagcttca 2160
   tcgacaacga agatgacgcc aagatcaact ggtctcccaa tggcgcaaca atgtatgctg 2220
   tggtaaacaa agacgagaag aacccctatg gagaaagccc cgggtacagg gttgcaccag 2280
   gttagtctca accgaacagg tgaaaaatac gacatcccga ctaacatcca aagcttctgg 2340
   tgtggcatat ctcacagtcc aggattcctc ggttatgcaa aacgccggtc accacacaac 2400
   gcatcatctc tacgccactc gacaaaagga caacgagctc tacgctgttg gagcgtataa 2460
   ttcgcttact cccgaggacc ctcaggttga ctttaatgag tatttcaaca gcgagtcgct 2520
   tgaccaagag gatatgtaag tattgtattt cccaccatgg catagacctg ggctgacttg 2580
   tgatacagtg ttctttggtt caacctggga atgcaccaca tgcctcacac aggtgatctt 2640
   cccaacaccg ttttctcaac cgcccactcc gctatgctca ttgagccatt caactatctt 2700
   atgggtgatc cttcgcaggc cagctctcag caagtcttaa tcaagactaa gaaggatggc 2760
   aagcctgaga ttgttactta tggcgctaag aatgcgacat gtgcgattga tatggtatgt 2820
   accttgatgt ctgtggtttt ttaccatgct aacatgactg attttaggca caactgaacc 2880
   ctgatctttc gaactattct aacagtatta gtgttctcaa gtaccccttc gatgggtcaa 2940
   agccagaccg tctttgaggc gcgcc 2965
<210> 11
   <211> 17
   <212> PRT
   <213> Artificial
<220>
   <223> Consensus motif
<220>
   <221> misc_feature
   <222> (4)..(4)
   <223> Xaa can be Asn or Ser
<220>
   <221> misc_feature
   <222> (14)..(14)
   <223> Xaa can be Ile or Leu
<400> 11
   Ile His Asp Xaa Leu Ser Gly Ser Met His Asp His Val Xaa Asn Phe
   1               5                   10                  15
   Lys

## Description

### Field of the invention

The present invention relates to lysyl oxidases.

### Background of the invention

### Lysyl oxidases

Amine oxidases form a heterogeneous family of enzymes that catalyze the oxidation of primary amine substrates to reactive aldehydes. The enzymes are subdivided into two groups on basis of the nature of their cofactor. Flavin adenine dinucleotide (FAD) is the cofactor of such enzymes as monoamine oxidase A and B and of an intracellular form of polyamine oxidase (Csiszar, Progr. Nucl Ac Res and Mol Biol (2001), 70, 1-32). A second group of amine oxidases contain a quinone, a modified tyrosine side chain, utilized as cofactor (Wang et al, Science (1996) 273, 1078-1084). Lysyl oxidase (LOX) enzymes belong to this subfamily of amine oxidases.

Amine oxidases catalyze the oxidative deamination of primary, secondary, and tertiary amines to the corresponding aldehydes, with the subsequent reduction of O₂ to H₂O₂ as illustrated below:

RCH₂NH₃⁺ + O₂ → RCHO + NH₄⁺ + H₂O₂

The amines found in mammals fall into the two groups mentioned above, flavin dependent monoamine oxidases (MAOs) and quinone-containing copper amine oxidases (CuAOs) (Duff et al, Biochem (2003) 42, 15148-15157). MAOs are found exclusively in the outer mitochondrial membrane of virtually all cell types and are involved in the degradation of primary, secondary and tertiary amines and the oxidation of several neurotransmitters. MAOs are believed to function either through a single electron-transfer or through a concerted covalent catalysis mechanism, both involving the FAD cofactor (Duff et al, Biochem (2003) 42, 15148-15157 and references cited therein). Quinone-containing CuAOs appear to be involved exclusively in the oxidative deamination of primary amines. These enzymes can be subdivided into two classes, based on the type of quinone cofactor present in the active site: topa quinone (TPQ) or lysyl tyrosylquinone (LTQ). Enzymes of the latter class, known as lysyl oxidases, have long been known to be associated with connective tissue formation through catalyzing the crucial deamination of the side chain of peptidyl lysine that initiates the cross-linking of lysine residues in collagen and elastin (Csiszar, Progr. Nucl Ac Res and Mol Biol (2001), 70, 1-32). While biogenesis of the LTQ-factor has yet to be examined, TPQ biogenesis has been extensively studied e.g. using multiple trapped crystal structures (Duff et al, Biochem (2003) 42, 15148-15157). The conversion of a tyrosine residue to TPQ requires only apo-unprocessed enzyme, copper, and molecular oxygen. The tyrosine that is modified is present in the conserved active site consensus sequence Asn-Tyr-Asp/Glu (Mu et al, J Biol Chem (1992) 267, 7979-7982).

Catalysis by CuAOs proceeds through a ping-pong mechanism (Wilmot et al, Science (1999) 286, 1724-1728). The key step is the conversion of the initial quinoneimine to quinoaldimine, facilitated by proton abstraction from the alpha-carbon of the substrate by an absolutely conserved aspartate acting a general base. Hydrolysis causes release of aldehyde product, giving a Cu(II)-aminoresorcinol in equilibrium with Cu(I)-semiquinone, which subsequently reacts with dioxygen to produce H₂O₂ and an iminoquinone. The later is then hydrolyzed, liberating NH₄⁺ and returning the cofactor in its resting state.

TPQ-containing CuAOs are distributed widely in nature and have been purified from mammals, plants and micro-organisms. They are involved in the oxidation of many short- to long-chained aliphatic mono- and diamines, including several aromatic amines (Duff et al, Biochem (2003) 42, 15148-15157). In micro-organisms they have a nutritional role in the utilization of primary amines as the sole source of nitrogen or carbon. The role of amine oxidases in higher organisms is not as clear. Several roles are proposed for these enzymes in plants and mammals such as wound healing, reduction of the concentration of toxic amines, cell growth, signaling and adhesion.

The induction of metalloproteins by metal ions has been observed in a wide range of animal tissues and microbial organisms (Rayton & Harris, J Biol Chem (1979) 254, 621-626). This principle was later used by Haywood and Large (Biochem J (1981) 199, 187-201) to help induce the expression of microbial amine oxidases during growth on media containing amines as sole nitrogen sources. They were able to induce the expression of two amine oxidases in *Candida boidinii.* The presence of two amine oxidases seems to be common in yeasts (Kuchar & Dooley, J Inorg Biochem (2001) 83, 193-204). They purified the corresponding enzymes and showed that they differed in substrate specificity. The enzymes were named methylamine oxidase and benzylamine oxidase. Later it was shown in a similar approach that the yeast *Pichia pastoris* also contains two amine oxidases (Green et al, Biochem J (1983) 211, 481-493). The benzylamine oxidase was further characterized by Tur & Lerch (FEBS Letters (1988) 238, 74-76). They demonstrated that the oxidase contains a quinone group, a copper atom and that the enzyme has a broad substrate specificity. Using lysine, model peptides, elastin and collagen as substrates they showed that the oxidase has substantial lysyl-oxidase activity. Based on inhibiton studies with β-aminopropionitril they suggested the enzyme is a copper lysyl-oxidase (E.C. 1.4.3.13). The enzyme has since then been referred to as lysyl oxidase. Dove et al (FEBS Letters (1996) 398, 231-234) showed that this copper lysyl oxidase contains TPQ as a cofactor, which distinguishes it from lysyl oxidases which are all mammalian and contain LTQ as cofactor. The enzyme was cloned, sequenced and further characterized by Kuchar & Dooley (J Inorg Biochem (2001) 83, 193-204). The were able to over express the intracellular enzyme to 10 mg per liter, making it the first lysyl oxidase that was successfully over expressed. They confirmed the presence of 1 copper ion per enzyme molecule and the presence of the TPQ cofactor. They further extended the work on the substrate specificity of the enzyme, showing that it is somewhat similar to mammalian oxidases and is able to oxidize lysine residues in peptides. The lysyl oxidase from *Pichia pastoris* has, however, a much broader substrate specificity compared to the mammalian enzymes (Kuchar & Dooley, J Inorg Biochem (2001) 83, 193-204). The enzyme shows rather low sequence homology to other amine oxidases. The highest homology was with human kidney diamine oxidase (50% similarity, 30% identity). A sequence comparison with other amine oxidases showed that only 29 residues were absolutely conserved, including those in the TPQ consensus sequence (Thr-Xxx-Xxx-Asn-Tyr-Glu/Asp-Tyr, in which Xxx is any of the twenty natural amino acids) and the three histidines that complex the copper atom.

The three dimensional structures have been determined of five amine oxidases, originating from *Escherichia coli* (Parsons et al, Structure (1995) 3, 1171-1184), *Pisum savitum* (Kumar et al, Structure (1996) 4, 943-955), *Hansenula polymorpha* (Li et al, Structure (1998) 6, 293-307), *Arthrobacter globiformis* (Wilce et al, Biochemistry (1997) 36, 16116-16133) and *Pichia pastoris* (Duff et al, Biochem (2003) 42, 15148-15157; Duff et al, Acta crystallogr D Biol crystallogr (2006) 62, 1073-1084). The *Pichia* enzyme is the only one that is a lysyl oxidase. Their structures are similar, showing four structural domains named D1, D2, D3 and D4 repsectively. The D1-domain has only been observed in the *E coli* enzyme, but is proposed to exist in the *Pichia*-enzyme as well. The active site is located in the large core domain D4, the smaller D3 and D2 domains are N-terminal to the D4 domain. The enzymes are crystallized as dimers. Unusual is the presence of a large solvent filled cavity, referred to as lake, in the interior of the dimer between the two D4 domains. The copper site in each subunit backs onto the lake with the TPQ cofactor lying on the distal side of the copper atom. A long and narrow channel connects the TPQ to the external solvent (Duff et al, Biochem (2003) 42, 15148-15157). This channel to the active site is particularly broad in the *Pichia* enzyme where is contains at its entrance a sequence of acidic residues Asp-Asp-Glu-Thr-Glu-Glu. It is thought that these residues facilitate the entrance of the charged lysine amino group via electrostatic interactions. Further down the channel it becomes more hydrophobic, facilitating the deprotonation of the lysine groups to the neutral amine form which can subsequently be oxidized in the active site. The active site comprises a copper atom coordinated by three histidines (res nr 528, 530 and 694)and by the 04 fo TPQ (res nr 478). The position of Asp398 in the active site is consistent with the proposed role of this residue as the totally conserved catalytic base.

For several years the lysyl oxidase from *Pichia pastoris* was the only known microbial lysyl oxidase. However, EP1 466 979 describes the finding of an amine oxidase derived from *Aspergillus oryzae* which is named lysyl oxidase. The genome of *Aspergillus oryzae* was used by the applicants to screen for lysyl oxidases, and they describe the finding of a gene that they characterize as highly homologous to the gene known from *Pichia pastoris.* It is unclear from the patent description what is considered highly homologous. We made a sequence alignment of the sequences from *Pichia pastoris* and *Aspergillus oryzae* and surprisingly found only 35% identity, which is generally not considered as a high homology. Percentages of 60%, preferably 70%, more preferably 80% and higher would be considered high homology. Furthermore, the lysyl oxidase from *Aspergillus oryzae* unexpectedly differs on several important positions from other lysyl oxidases and amine oxidases: e.g. position 164 of the annotated protein of the *Asperillus oryzae* gene described in EP 1 466 979 (SEQ_ID NO: 2) is an alanine, while in all other described amine and lysyl oxidases this position is a highly conserved proline. Additionally position 78 in this annotated protein is a methionine, while leucine is highly conserved in amine oxidases at this position. Furthermore, the protein described in EP 1 466 979 contains an extra insertion of 2 amino acids after residue 109 when compared to mammalian and *Pichia* lysyl oxidase. Also position 499 in the protein described in EP 1 466 979 is a threonine, while this position is always an hydrophobic residue in other amine oxidases. Conserved positions in proteins are often associated with the primary function of an enzyme, and cannot be changed without changing the catalytic activity of the enzyme. It is remarkable that in this particular lysyl oxidase several of such conserved residues are modified, and they are expected to have an effect on enzyme activity.

The gene was cloned in *Aspergillus nidulans* via random integration of the gene in the genome. The applicants indicate that instead of *A. nidulans,* also other strains could be used such as *Aspergillus niger* and *Aspergillus oryzae.* Surprisingly, it is described in EP1 466 979 that lysyl oxidase activity can be measured in the culture fluid of some transformants when this gene was over expressed, in contrast to the *Pichia pastoris* enzyme which is an intracellular enzyme. Apparently, the lysyl oxidase from *Aspergillus oryzae* is an secreted enzyme. The presence of the enzyme was established via activity measurements. For the characterization of lysyl oxidases (E.C. 1.4.3.13) and their distinction from lysine oxidases (E.C. 1.4.3.14) the use of correct substrates is of crucial importance. The assay described for lysyl oxidases in EP1 466 979 (example 11 in that patent) uses lysine as the sole substrate. This amino acid has two free amino groups, one at the C^{α} atom and one in the lysine side chain at the C^{ε} atom. Lysyl oxidases only oxidize the latter and leave the other group unmodified. This can be determined by using lysine substrates in which the α-amino group is blocked by e.g. acetylation. Such substrates are essential for proper identification of lysyl oxidases, and are very commonly used in the characterization of such enzymes (see e.g. Kuchar & Dooley, J Inorg Biochem (2001) 83, 193-204). Since such substrates are not used in EP1 466 979 it is not possible to conclude from the data, provided in EP1 466 979, that the enzyme activity found is indeed lysyl oxidase activity. The enzyme could well be a lysine oxidase (E.C. 1.4.3.14; L-lysine: oxygen 2-oxidoreductase) and not a lysyl oxidase (E.C. 1.4.3.13, protein-L-lysine: oxygen 6-oxidoreductase). Also, the measured activity was rather low, being only a few times the background activity of the non-transformed strain (EP1 466 979, example 11). In order to clarify this matter we tried to over-express the protein of SEQ_ID NO: 2 (EP 1 466 979) in *Aspergillus niger* (example 1 of the current application). According to EP1 466 979, *Aspergillus niger* is a preferred expression organism for the lysyl oxidase. We were not able to isolate or otherwise demonstrate an active lysyl oxidase from the culture fluid of all transformants tested. In view of the arguments discussed above, in combination with the experimental work described in examples 1 and 2 of the current application, it is unlikely that EP1 466 979 described the identification of a functional lysyl oxidase. EP1 466 979 also described no attempt to purify the enzyme but instead activity measurements are performed with crude fermentation supernatants that may contain a variety of unspecified enzyme activities. Additionally, not all transformants described in EP 1 466 979 showed increased activity compared to the non-transformed strain. It is therefore doubtful if the protein sequence described in EP 1 466 979 is indeed correct and codes for a functional lysyl oxidase. The oxidase activity that has been measured in some of the transformants in EP 1 466 979 might therefore not be due to the over expression of the described gene but might be caused by the presence of background amine oxidase activity. It is surprising that in the medium of the reference strain, a considerable amount of arginine is added (EP1 466 979, example 11). It is known that amine oxidases can be inhibited by guanidine containing compounds, such as arginine (Kuchar & Dooley, J Inorg Biochem (2001) 83, 193-204), and arginine contains such guanidine group. In case the empty strain would produce low amounts of amine oxidase activity, this might be inhibited by the arginine that is present in the medium. If this is the case, the empty strain would be tested in a set-up that was not a good control since the back ground would be artificially lowered. In that situation, it could be the reason for the reported low activity of the transformants, which showed only a few times the background activity. This low activity might be the regular back ground activity observed in absence of arginine.

From all the above it is clear that lysyl oxidases are known, but no lysyl oxidase has been produced extra cellular. The *Pichia* lysyl oxidase is an intracellular enzyme and is only produced at levels of 10 mg/ml (Kuchar & Dooley, J Inorg Biochem (2001) 193-204). Furthermore, the enzyme has to be released from the cell, which makes the processing of the enzyme at an industrial scale complicated and unattractive. Since also other lysyl oxidases have not been overproduced to economically interesting expression levels there is still a need for an extra cellular lysyl oxidase that can be produced in high quantities (grams per liter).

From an economic point of view there exists a clear need for an improved means of producing lysyl oxidases in high quantities and in a relatively pure form, compared to the poor productivity of the *Pichia pastoris* enzyme and the alleged *Aspergillus oryzae* enzyme. A preferred way of doing this is via the overproduction of such a lysyl oxidase using recombinant DNA techniques. A particularly preferred way of doing this is via the overproduction of a fungal derived lysyl oxidase and a most preferred way of doing this is via the overproduction of an *Aspergillus* derived lysyl oxidase. To enable the latter production route unique sequence information of an *Aspergillus* derived lysyl oxidase is essential. More preferable the whole nucleotide sequence of the encoding gene has to be available.

An improved means of producing the newly identified secreted lysyl oxidase in high quantities and a relatively pure form is via the overproduction of the *Aspergillus* encoded enzyme using recombinant DNA techniques. A preferred way of doing this is via the overproduction of such a secreted lysyl oxidase in a food grade host microorganism. Well known food grade micro organisms include *Aspergilli, Trichoderma, Streptomyces, Bacilli* and yeasts such as *Saccharomyces* and *Kluyveromyces.* An even more preferred way of doing this is via overproduction of the secreted *Aspergillus* derived lysyl oxidase in a food grade fungus such as *Aspergillus.* Most preferred is the over production of the secreted lysyl oxidase in a food grade fungus in which the codon usage of the lysyl oxidase-encoding gene has been optimized for the food grade expression host used. In general, to enable the latter optimization routes, unique sequence information of a secreted lysyl oxidase is desirable. More preferable the whole nucleotide sequence of the lysyl oxidase encoding gene has to be available. Once the gene encoding a secreted lysyl oxidase is transformed in a preferred host, selected strains can be used for fermentation and isolation of the secreted lysyl oxidase protein from the fermentation broth.

Once the new enzyme has been made available in large quantities and in a relatively pure form, food proteins or hydrolysates with improved texture are producible in a food grade and economic way.

Enzymes that can catalyze the formation of covalent protein cross links are rare. An example is the class of protein sulfhydryl oxidases, which can catalyze the formation of disulfide bonds between proteins. The applicability of these enzymes is, however, limited primarily because of the limited occurrence of free sulfhydryl groups in proteins. Especially food proteins are frequently subject to oxidizing conditions during processing (e.g. during drying), which often leads to oxidation of available thiol groups. In some applications, however, such as bread, disulfide bond formation and disulfide bond reshuffling is of high relevance for proper texture formation.

Another well known class of protein cross linking enzymes is transglutaminase, which can catalyze the formation of a covalent cross-link between proteins via a lysine and a glutamine residue. Transglutaminase (E.C. 2.3.2.13) catalyzes an acyl-transfer reaction in which the γ-carboxamide groups of peptide bound glutamine residues are the acyl donors. Microbial transglutaminase, derived from *Streptoverticillium mobaraense* is commercially available under the trade name Activa TG (Ajinomoto, Japan), but also blood-derived transglutaminase is commercially available (trade name: Harimex). Transglutaminase has been used to catalyze the cross-linking of whey proteins, soy proteins, wheat proteins, beef myosin, casein and crude actomyosin refined from mechanically deboned poultry meat, (Zhu et al, Appl Microbiol Biotechnol (1995) 44, 277-282). Use of transglutaminase has also been described for fish products, meat substitutes and alternatives, (processed) cheese products, boiled rice, noodles, yogurt and (frozen) dairy products. Examples of transglutaminase applications are described in the following patents: JP09206006 (boiled rice), JP09154512 (noodles), JP092060031 (fish), JP08224063 (protein gelling), JP08112071 (Tofu), JP08056597 (good crispness after frying), JP07184554 (ice cream), WO 9520662 A (Japanese oyster), JP06261712 (crab), EP 610649 (yogurt), JP06153827 (rice), WO9319610 (milk), JP02131537 (cheese). Often the purpose is to modify food texture, but it is also used to e.g. increase cheese yield. A disadvantage of (microbial) transglutaminase is its relatively high heat stability, which makes it difficult to inactivate completely under regular food processing conditions, such as pasteurization, sterilization or ultra-high heat treatment. At 70°C, the enzyme inactivation requires 15 minutes and at 75°C this is still 5 minutes (product information sheet Activa TG, Ajinomoto). Normal pasteurization times are 15-30 seconds at 72°C, which is much shorter than the time required for inactivation of transglutaminase. Inactivation of the transglutaminase enzyme is required because of food regulations. Long heating at elevated temperatures is undesired for many food applications because it leads to the formation of undesired off flavors and off colors resulting from e.g. Maillard reactions. Several other enzyme classes have been described as usefull to obtain protein cross-linkers, including lipoxygenase, proteindisulfideisomerase, phenoloxidase, peroxidases, proteindisulfide reductases, and tyrosin oxidases. Exampels of substrates for protein cross linking enzymes include all proteins derived from animal or plant sources, e.g. gelatin, milk proteins such as whey and casein, soy protein, wheat protein, gluten, maize protein, pea protein and collagen.

A few patents describe the use of lysyl oxidases. JP02245166 and JP04094670 describe the use of lysyl oxidase to modify fish protein for fish paste. DE1940069 describes the manufacture of a cross linked protein coating for active substances in which lysyl oxidase is used to obtain protein cross linking. JP007825 describes the preparation of protein sheet molding material in which lysyl oxidase in mentioned as a possible way to cross link proteins. WO200307728 describes the preparation foodstuff for vegetarians from enzymatically cross-linked fungal protein, in which transglutaminase, protein disulphide isomerase, sulphydryl oxidase, several polyphenyl oxidases, lysyloxidase, peroxidase and lipoxygenase are mentioned as possible cross-linking enzymes or fungal protein. WO2004105485 describes the preparation of slow release particles in which the possible use of lysyl oxidase for protein cross linking is mentioned. These examples demonstrate and support the industrial use of a lysyl oxidase.

### Summary of the invention

It has surprisingly been fond that a single combination of 5 amino acid sequence motifs is able to identify novel class of excreted microbial lysyl oxidases with unique properties. The class of lysyl oxidases described herein is unique because they are excreted and because they have a preference for amino groups in lysyl residues in peptides or proteins over amino groups in low molecular weight substrates such as benzyl amine and tryptamine.

Herein is described:
(1) Lysyl oxidases of *Aspergillus nidulans, Cryptococcus neoformans, Podospora anserina* and *Fusarium graminearum;*
(2) Lysyl oxidases that contain the following amino acid motif (residues in brackets indicate allowed degeneracy at that point, amino acids are in 1-letter code): I-H-D-[NS]-L-S-G-S-M-H-D-H-V-[IL]-N-F-K (SEQ_ID NO:11)
(3) a DNA sequence encoding a lysyl oxidase of (1) or (2);
(4) an expression vector comprising a DNA sequence of (3);
(5) a host cell comprising an expression vector of (4); and
(6) a process for identifying novel texture and yield improving enzymes comprising screening and amino acid sequence for the presence of the motif from (2).
(7) A process to prepare reactive protein intermediates that can be activated by a heating step to form a protein gel.

The lysyl oxidases described herein are able to catalyze the oxidative deamination of lysyl groups in a protein or peptide to the corresponding aldehydes, with the subsequent reduction of O₂ to H₂O₂ and are advantageously used in the preparation of food, feed or nutraceutical products, or intermediate products thereof.

A lysyl oxidase suitable for use in the invention is able to catalyze the oxidative deamination of lysyl groups in a protein or peptide to the corresponding aldehydes, with the subsequent reduction of O₂ to H₂O₂ whereby the lysyl oxidase
(a) shows a ratio of between lysyl oxidase activity for Ac-Gly-Lys-OMe and lysyl oxidase activity for benzylamine of at least 1.1, preferably at least 1.3, more preferably of at least 1.4 whereby the activities are determined at 37 degrees Celsius and an pH of 7.0; and.
(b) has an optimum activity at a temperature of between 0 and 60 degrees Celcius, and the prefered use thereof in the preparation of food, feed or nutraceutical products, or intermediate products thereof.

Accordingly the invention provides use of a polypeptide which has lysyl oxidase activity and which comprises the following amino acid motif, residues in brackets indicate allowed degeneracy at that point, amino acids are in 1-letter code, :
- I H D [N S] L S G S M H D H V [IL] N F K
to catalyze the oxidative deamination of lysyl groups in a protein or petide to the corresponding aldehydes, with the with the subsequent reduction of O₂ to H₂O₂.

Such a polypeptide may be used in the preparation of food, feed or nutraceutical products, or intermediate products thereof.

To another aspect of the invention use of a polypeptide is provided which has lysyl oxidase activity, selected from the group consisting of:
(a) a polypeptide which has an amino acid sequence which has at least 60% amino acid sequence identity with the whole amino acid sequence of any one of SEQ ID NO: 1 to 4;
(b) a polypeptide which is encoded by a polynucleotide which hybridizes under low stringency conditions with (i) the nucleic acid sequence of SEQ ID NO: 6 or (ii) a nucleic acid sequence complementary to the nucleic acid sequence of SEQ ID NO: 6.

The lysyl oxidase is advantageously used for the preparation of a food or feed or a nutraceutical or for the preparation of an intermediate product for the preparation of a food or feed or a nutraceutical.

According to a further aspect of the invention a method is provided for the production of lysyl oxidase which comprises cultivating a host cell comprising a nucleic acid construct comprising a polynucleotide encoding the lysyl oxidase which comprises the amino acid sequence:
- I H D [N S] L S G S M H D H V [IL] N F K;
under conditions suitable for production of the lysyl oxidase; and recovering the lysyl oxidase.

The present invention also relates to a process for identifying new lysyl oxidases comprising screening an amino acid sequence for the presence of the amino acid sequences:
- I H D [N S] L S G S M H D H V [IL] N F K.

Furthermore the invention provides a process to modify a food or feed product which contains a protein or peptide, said method comprising modifying the food or feed product by contacting it with a lysyl oxidase as described herein or an enzyme composition comprising the lysyl oxidase described herein.

The one-letter code of amino acids used herein is commonly known in the art and can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989).

An aspect of the present invention is the over-expression of secreted lysyl oxidases when the sequences described herein are used. Furthermore, we provide a sequence motif which can be used to identify and select active secreted lysyl oxidases from different fungi. Using this approach we were able to select and express lysyl oxidases from *Aspergillus nidulans, Fusarium graminearum, Podospora anserina* and *Cryptococcus neoformans.*

The lysyl oxidases will be inactivated during pasteurization processes regularly used in the food industry.

The pasteurisation treatment may be performed at a temperature of at least 75°C, preferably at least 80°C. In one embodiment the heat treatment is conducted at a temperature between 75°C and 145°C, in a preferred embodiment the heat treatment is conducted at a temperature between 75°C and 120°C, in a more preferred embodiment the heat treatment is conducted at a temperature between 75°C and 100°C, in an even more preferred embodiment the heat treatment is performed between 80°C and 90°C. The duration of the heat treatment may be any time suitable to achieve the deactivation of the lysyl oxidase. In one embodiment the duration of the heat treatment is between 1 second and 30 minutes. In one embodiment the heat treatment is conducted at 75°C to 90°C degrees for 5 seconds to 30 minutes, in another embodiment the heat treatment is conducted at 80°C to 90°C for 2 seconds to 30 minutes, in a still further embodiment the heat treatment is conducted at 80°C to 145°C from 1 second to 20 minutes. The heat treatment may be conducted by any method known in the art.

Accordingly the enzyme described herein, when incubated with proteins, leads to modification in texture of the protein gels. The modification is obtained in a novel two step process. In the first step the protein is treated with the lysyl oxidase. The cross linking is obtained in a second step in which the protein is heated to at least 75°C, preferably 80°C. Prior to cross linking, the protein may optionally be ultrafiltrated, or dried under temperature conditions not leading to substantial (<1%) protein cross linking. Drying temperatures are preferably below 60°C, more preferably below 50°C , most preferably below 40°C. The heat treatment may coincide with the heat treatment used to inactivate the enzyme. The resulting cross linked protein may be used as an ingredient in food and feed and improve their texture.

According to another aspect of the invention the enzyme described herein, when incubated with proteins, leads to modification in texture of the protein gels.

The present invention also provides a process for the preparation of a reactive aldehyde in a protein or peptide which comprises the oxidative deamination of lysyl-amine of the protein or peptide by a lysyl oxidase and preferably concentrating and/or drying the protein or peptide containing the reactive aldehydes.

Furthermore the present invention provides a process for the preparation of a cross-linked protein or peptide which comprises treating, preferably by heating, protein or peptide which comprises reactive aldehydes, to cross-link the protein or peptide.

A protein or peptide which comprises reactive aldehydes formed by the oxidative deamination of lysyl-amine of the protein, is preferably in dried form.

### Detailed description of the invention

A "peptide" or "oligopeptide" is defined herein as a chain of at least two amino acids that are linked through peptide bonds. The terms "peptide" and "oligopeptide" are considered synonymous (as is commonly recognized) and each term can be used interchangeably as the context requires.

A "polypeptide" is defined herein as a chain comprising of more than 30 amino acid residues. All (oligo)peptide and polypeptide formulas or sequences herein are written from left to right in the direction from amino-terminus to carboxy-terminus, in accordance with common practice. The one-letter code of amino acids used herein is commonly known in the art and can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989).
By milk protein is meant milk, skim milk, fat free milk, butter milk, yoghurt, milk powder dissolved in water to the desired protein concentration, caseinate dissolved in water to the desired protein concentration optionally containing whey proteins, a solution of glycomacropeptide (GMP) from kappa-casein or a combination thereof.
By hydrolysate is meant the product that is formed by the hydrolysis of the protein (or briefly protein hydrolysate or hydrolysed protein), the acid-soluble hydrolysate being the soluble fraction of the protein hydrolysate which is also described herein as soluble peptide containing composition or composition comprising soluble peptides), or a mixture of a protein hydrolysate and an acid soluble hydrolysate.

The term nutraceutical as used herein denotes the usefulness in both the nutritional and pharmaceutical field of application. Thus, novel nutraceutical compositions comprising the composition of the invention can find use as supplement to food and beverages and as pharmaceutical formulations or medicaments for enteral or parenteral application which may be solid formulations such as capsules or tablets, or liquid formulations, such as solutions, suspensions or emulsions.

Suitable substrates for the cross-linking according to the invention are proteins, peptides, hydrolysates, modified proteins or peptides and other compounds having an amine-functionality.

The cross-linking enzymes that are described above establish covalent bonds between protein molecules. The enzyme transglutaminase catalyzes bond formation directly using glutamine and lysine residues. The effects of transglutaminase action are often immediately visible, e.g. as an increase in viscosity or an improved and firmness (improved texture). (see e.g. Technical information by Ajinomoto: Activa®WM, Das enzym mit biss; Thought TNO newletter, TNO Research Institute, Netherlands, No 25, October 1998, page 3) and do not require further processing steps to obtain the crosslinks. Other enzymatic crosslinking strategies using e.g. sulfhydrolyoxidase or hydrogenperoxide generating enzymes also lead to protein cross-linking without requiring further processing steps. The same is claimed for lysyl oxidases, which generate a reactive aldehyde function by their action on lysine residues in proteins, and application of these modified proteins has been described, e.g. in EP 0988859. In this patent application the use of lysyl oxidase to obtain protein cross linking is described. The authors specifically indicate that the modified protein should be treated at temperatures below 80°C, preferably below 60°C. Apparently, heat treatment is not necessary to obtain protein linking. Solution of cross linked proteins often show increased viscosity and increased water binding capacity, compared to the non-cross linked proteins under similar conditions. This is a disadvantage when the cross-linked proteins needs to be further processed before shipping it from the ingredients supplier to the users like food manufacturers. The cross-linked protein is more difficult to handle and more difficult to process (e.g. filtration) because of the high viscosity of the solution containing the cross linked protein; also other processing steps like e.g. drying become more difficult and expensive when handling cross-linked proteins. The drying process takes of the cross linked proteins takes more energy compared to that of the not cross linked proteins because it has better water binding capacity. It would be of interest when reactive protein intermediates could be prepared from the starting protein substrate that are easily cross in the application when desired. It would prevent the formation of viscous and high water binding preparations at the site of the ingredients manufacturer, preventing excessive operating costs and handling. Reactive protein intermediates require the modification (functionalization) of the starting protein substrates. In this patent application we show that surprisingly proteins modified with the lysyl oxidases described in this application have characteristics of such reactive protein intermediates. Proteins such as whey and casein proteins that are modified with lysyl oxidase do not show increased viscosity. When the functionalized proteins are subsequently heated, the reactive protein intermediates form protein cross links, leading to increased viscosity of protein solutions and increased texture.

Several lysyl oxidases have been described in literature, as indicated above. Most of them are from mammalian origin, and have not been over expressed at high levels due to their poor solubility characteristics (Kuchar & Dooley, J Inorg Biochem (2001) 83, 193-204). Also the lysyl oxidase from *Pichia pastoris* is poorly expressed (10 mg/L, Kuchar & Dooley, J Inorg Biochem (2001) 83, 193-204), and this enzyme is also intracellular produced. EP 1 466 979 describes an alleged lysyl oxidase, but we have argued above why we believe this is not a lysyl oxidase. In addition we demonstrate in example 1 that the cloning and expression of the lysyl oxidase described in EP 1 466 979 (SEQ_ID NO: 2 in that patent) does not lead to production of lysyl oxidase activity.

We have surprisingly found that polypeptides containing the amino acid sequence motif SEQ_ID NO:11 code for active fungal lysyl oxidases that are secreted from the cell in the culture medium. We provide four examples of such lysyl oxidases. The sequence of lysyl oxidase ZGR is obtained from *Aspergillus nidulans* (SEQ_ID NO 1 and 6 coding for the amino acid sequence and a synthetic gene encoding this protein respectively); lysyl oxidase ZGT is obtained from *Cryptococcus neoformans* (SEQ_ID NO 2 and 7 coding for the amino acid sequence and a synthetic gene encoding this protein respectively); lysyl oxidase GLO1 is obtained from *Fusarium graminearum* (SEQ_ID NO 3 and 8 coding for the amino acid sequence and the genomic DNA sequence encoding this protein respectively); lysyl oxidase ZGY is obtained from *Podospora anserina* (SEQ_ID NO 4 and 9 coding for the amino acid sequence and a synthetic gene encoding this protein respectively). Surprisingly, a polypeptide that is highly homologous to these four proteins but that did not contain the amino acid sequence motif of SEQ_ID NO:11 was also produced, but did not show the desired enzymatic activity, in contrast to the four proteins that did contain the amino acid sequence motif of SEQ_ID NO:11. This amine oxidase GLO2 is obtained from *Fusarium graminearum* (SEQ_ID NO 5 and 10 coding for the amino acid sequence and the genomic DNA sequence encoding this protein respectively). The four lysyl oxidases that were identified using the amino acid sequence motif SEQ_ID NO:11 had higher preference for the lysine substrates, and lysyl oxidases that lack the motif SEQ_ID NO:11 have a less preferred activity spectrum. Apparently the search with the amino acid motif SEQ_ID NO:11 identifies specifically the lysyl oxidases with more preferred activity on lysine substrates, and is therefore an important new tool in the identification of useful lysyl oxidases.

The motif SEQ_ID NO:11 is located at the active centre of the lysyl oxidase, when it is superimposed on the known protein structure of the *Pichia pastoris* lysyl oxidase (Duff et al. (2003) Biochemistry 42, 15148-15157). The two histidines in this motif correspond to H528 and H530 of the *Pichia pastoris* lysyl oxidase protein. These two histidines are involved in the binding of the copper atom at the active site of the enzyme. It is very well conceivable that differences in this amino acid motif lead to changes close or at the active centre of the enzyme, and therefore have an effect on the activity or substrate specificity of the enzyme. We propose that using this motif a person skilled in the art can identify active fungal lysyl oxidases that are useful for the application in food and feed.

The ability of the enzymes described herein to oxidase lysyl residues in peptides is of interest for industrial applications. Even more advantageous is that the present enzymes are able to oxidize lysyl residues in proteins. Lysyl oxidases which have a high preference for lysyl residues in proteins and peptides compared to low molecular weight substrates like free lysine or benzyl amine are therefore preferred in the industry. This substrate preference is an important advantegous and parameter for the selection of lysyl oxidases for industrial use.

We show in this invention that it is indeed possible to over-express secreted lysyl oxidases with the proper substrate specificity when the correct sequences are used. Furthermore, we provide a sequence motif which can be used to identify and select active secreted lysyl oxidases from different fungi. Using this approach we were able to select and express lysyl oxidases from *Aspergillus nidulans, Fusarium graminearum, Podospora anserina* and *Cryptococcus neoformans.*

Lysyl-oxidases catalyze the oxidative deamination of lysyl groups in proteins:

R₁-(CH₂)₄-NH₂ + O₂ → R₁-(CH₂)₃-CHO + H₂O₂ + NH₃

In this reaction, R₁ is the protein chain which the lysine residue is part of. The reactive aldehyde can react with a free amino group of a second lysine residue:

R₁CH₂)₃-CHO + R₂-(CH₂)₄-NH₂ → R₁-(CH₂)₃-NH-(CH₂)₄-R₂ + H₂O

In this way a covalent bond between two protein molecules is formed. Instead of with a second lysine residue, the reactive aldehyde can also react with available amine groups other than those from protein lysine residues to form a covalent bond. The cross linking of proteins is especially useful in modifying the texture of foods. Foods are multi component materials with complex structures and textures that are appreciated by consumers. A major task of modern food technologists is to generate new engineered structures from a limited range of ingredients that have acceptable taste and texture characteristics (Dickinson, Trends Food Sci technol (1997) 8, 334-339). Proteins are one of the main classes of building blocks that are available to the food technologist for conferring semisolid textural attributes, and the cross-linking and aggregation of protein molecules into three-dimensional networks (gels) is one of the most important mechanisms for developing structures with desirable mechanical properties. Among the many traditional food textures that are based predominantly on protein gels are those of cheese, yoghurt, sausage, tofu (soybean curd) and surimi (fish meat gel). Several interactions play a role in gel formation, such as hydrophobic forces, electrostatic interactions, hydrogen bonds and covalent interactions. The latter lead to permanent cross links in the gel network. The visco-elastic and fracture properties of a food gel, and hence its texture as perceived by consumers, are dependent on the balance between the gel stabilizing forces, including the covalent interactions. Since lysyl-oxidases can be used for the formation of intramolecular covalent bonds they are useful for modification of food texture. We found that the enzyme, when incubated with proteins, leads to modification in texture of the protein gels. Moreover there is an industrial need for protein cross-linking enzyme that is inactivated during pasteurization processes regularly used in the food industry. We show that the lysyl oxidases described herein fulfill the criteria of heat lability.

A polypeptide which has lysyl oxidase activity may be in an isolated form. As defined herein, an isolated polypeptide is an endogenously produced or a recombinant polypeptide which is essentially free from other non-lysyl oxidase polypeptides, and is typically at least about 20% pure, preferably at least about 40% pure, more preferably at least about 60% pure, even more preferably at least about 80% pure, still more preferably about 90% pure, and most preferably about 95% pure, as determined by SDS-PAGE. The polypeptide may be isolated by centrifugation and chromatographic methods, or any other technique known in the art for obtaining pure proteins from crude solutions. It will be understood that the polypeptide may be mixed with carriers or diluents which do not interfere with the intended purpose of the polypeptide, and thus the polypeptide in this form will still be regarded as isolated. It will generally comprise the polypeptide in a preparation in which more than 20%, for example more than 30%, 40%, 50%, 80%, 90%, 95% or 99%, by weight of the proteins in the preparation is a polypeptide for use in the invention.

Preferably, the polypeptide used in the invention is obtainable from a microorganism which possesses a gene encoding an enzyme with lysyl oxidase activity. More preferably the polypeptide is secreted from this microorganism. Even more preferably the microorganism is fungal, and optimally is a filamentous fungus. Preferred donor organisms are thus of the genus *Fusarium,* such as those of the species *Fusarium graminearum,* of the genus *Aspergillus,* such as those of the species *Aspergillus nidulans,* of the genus *Cryptococcus,* such as those of the species *Cryptococcus neoformans,* or of the genus *Podospora,* such as those of the species *Podospora anserina.*

The present invention provides use of an isolated polypeptide having an amino acid sequence which has a degree of amino acid sequence identity to amino acids 1 to 817 of SEQ_ID NO:1 (i.e. the polypeptide) of at least 30%, preferably at least 40%, preferably at least 50%, preferably at least 60%, preferably at least 70%, more preferably at least 80%, even more preferably at least 90%, still more preferably at least 95%, and most preferably at least 97%, and which has lysyl oxidase activity.

For the purposes of the present invention, the degree of identity between two or more amino acid sequences is determined by BLAST P protein database search program (Altschul et al., 1997, Nucleic Acids Research 25: 3389-3402) with matrix Blosum 62 and an expected threshold of 10.

A polypeptide used in the invention may comprise the amino acid sequence set forth in SEQ_ID NO:1 or a substantially homologous sequence, or a fragment of either sequence having lysyl oxidase activity. In general, the naturally occurring amino acid sequence shown in SEQ_ID NO:1 is preferred.

The polypeptide used in the invention may also comprise a naturally occurring variant or species homologue of the polypeptide of SEQ_ID NO:1.

A variant is a polypeptide that occurs naturally in, for example, fungal, bacterial, yeast or plant cells, the variant having lysyl oxidase activity and a sequence substantially similar to the protein of SEQ_ID NO:1. The term "variants" refers to polypeptides which have the same essential character or basic biological functionality as the lysyl oxidase of SEQ_ID NO: 1, and includes allelic variants. Preferably, a variant polypeptide has at least the same level of lysyl oxidase activity as the polypeptide of SEQ_ID NO: 1. Variants include allelic variants either from the same strain as the polypeptide of SEQ_ID NO: 1. or from a different strain of the same genus or species.

Similarly, a species homologue of the inventive protein is an equivalent protein of similar sequence which is an lysyl oxidase and occurs naturally in another species. Examples of species homologues of the polypeptide of SEQ_ID NO: 1 are listed in SEQ_ID NO: 2, 3 and 4.

Variants and species homologues can be isolated using the procedures described herein which were used to isolate the polypeptide of SEQ_ID NO: 1 and performing such procedures on a suitable cell source, for example a bacterial, yeast, fungal or plant cell. Also possible is to use a probe to probe libraries made from yeast, bacterial, fungal or plant cells in order to obtain clones expressing variants or species homologues of the polypepetide of SEQ_ID NO: 1. The methods that can be used to isolate variants and species homologues of a known gene are extensively described in literature, and known to those skilled in the art. These genes can be manipulated by conventional techniques to generate a polypeptide for use in the invention which thereafter may be produced by recombinant or synthetic techniques known *per se.*

The sequence of the polypeptide of SEQ_ID NO: 1 and of variants and species homologues can also be modified to provide polypeptides for use in the invention. Amino acid substitutions may be made, for example from 1, 2 or 3 to 10, 20 or 30 substitutions. The same number of deletions and insertions may also be made. These changes may be made outside regions critical to the function of the polypeptide, as such a modified polypeptide will retain its lysyl oxidase activity.

Polypeptides for use in the invention include fragments of the above mentioned full length polypeptides and of variants thereof, including fragments of the sequence set out in SEQ_ID NO: 1. Such fragments will typically retain activity as an lysyl oxidase. Fragments may be at least 50, 100 or 200 amino acids long or may be this number of amino acids short of the full length sequence shown in SEQ_ID NO: 1.

Polypeptides for use in the invention can, if necessary, be produced by synthetic means although usually they will be made recombinantly as described below. Synthetic polypeptides may be modified, for example, by the addition of histidine residues or a T7 tag to assist their identification or purification, or by the addition of a signal sequence to promote their secretion from a cell.

Thus, the variants sequences may comprise those derived from strains of *Aspergillus* other than the strain from which the polypeptide of SEQ_ID NO: 1 was isolated. Variants can be identified from other *Aspergillus* strains by looking for lysyl oxidase activity and cloning and sequencing as described herein. Variants may include the deletion, modification or addition of single amino acids or groups of amino acids within the protein sequence, as long as the peptide maintains the basic biological functionality of the lysyl oxidase of SEQ_ID NO: 1.

Amino acid substitutions may be made, for example from 1, 2 or from 3 to 10, 20 or 30 substitutions. The modified polypeptide will generally retain activity as a lysyl oxidase. Conservative substitutions may be made; such substitutions are well known in the art.

Shorter polypeptide sequences are within the scope of the invention. For example, a peptide of at least 50 amino acids or up 100, 150, 200, 300, 400, 500, 600, 700 or 800 amino acids in length is considered to fall within the scope of the invention as long as it demonstrates the basic biological functionality of the lysyl oxidase of SEQ_ID NO: 1. In particular, but not exclusively, this aspect of the invention encompasses the situation in which the protein is a fragment of the complete protein sequence.

The present invention also describes a polynucleotide which encodes a polypeptide which has lysyl oxidase activity, said polynucleotide comprises
(a) a polynucleotide sequence which encodes amino acid SEQ_ID NO:1, and
(b) a polynucleotide sequence which encodes amino acid SEQ_ID NO:2, and
(c) a polynucleotide sequence which encodes amino acid SEQ_ID NO:3, and
(d) a polynucleotide sequence which encodes amino acid SEO_ID NO:4.

For the present invention polypeptides that contain the lysyl oxidase consensus sequences of SEQ_ID NO: 11 are within the invention. For the present invention it is especially relevant that the protein of interest is actively secreted into the growth medium. Secreted proteins are normally originally synthesized as pre-proteins and the pre-sequence (signal sequence) is subsequently removed during the secretion process. The secretion process is basically similar in prokaryotes and eukaryotes: the actively secreted pre-protein is threaded through a membrane, the signal sequence is removed by a specific signal peptidase, and the mature protein is (re)-folded. Also for the signal sequence a general structure can be recognized. Signal sequences for secretion are located at the amino-terminus of the pre-protein, and are generally 15-30 amino-acids in length. The amino-terminus preferably contains positively charged amino-acids, and preferably no acidic amino-acids. It is thought that this positively charged region interacts with the negatively charged head groups of the phospholipids of the membrane. This region is followed by a hydrophobic, membrane-spanning core region. This region is generally 10-20 amino-acids in length and consists mainly of hydrophobic amino-acids. Charged amino-acids are normally not present in this region. The membrane spanning region is followed by the recognition site for signal peptidase. The recognition site consists of amino-acids with the preference for small-X-small. Small amino-acids can be alanine, glycine, serine or cysteine. X can be any amino acids. Using such rules an algorithm has been written that is able to recognize such signal sequences from eukaryotes and prokaryotes (Bendtsen, Nielsen, von Heijne and Brunak. (2004) J. Mol. Biol., 340:783-795). The SignalP program to calculate and recognize signal sequences in proteins is generally available (http://www.cbs.dtu.dk/services/SignalP/).

Relevant for the present invention is that signal sequences can be recognized from the deduced protein sequence of a sequenced gene. If a gene encodes a protein where a signal sequence is predicted using the SignalP program, the chance that this protein is secreted is high. Preferably, the purpose of this invention is therefore to provide a new method to find new proteins that have lysyl oxidase activity using the consensus of SEQ_ID NO: 11, in combination with the presence of a signal sequence detected by the SignalP program.

In a second embodiment, the present invention provides use of an isolated polypeptide which has lysyl oxidase activity, and is encoded by poly-nucleotides which hybridize or are capable of hybrizing under low stringency conditions, more preferably medium stringency conditions, and most preferably high stringency conditions, with (i) the nucleic acid sequence of SEO_ID NO:6 or (ii) a nucleic acid fragment comprising at least a portion of SEQ_ID NO:6, or (iii) having bases differing from the bases of SEQ_ID NO:6; or (iv) with a nucleic acid strand complementary to SEQ_ID NO: 6.

The term "capable of hybridizing" means that the target polynucleotide can hybridize to the nucleic acid used as a probe (for example, the nucleotide sequence set forth in SEQ_ID NO: 6, or a fragment thereof, or the complement of SEQ_ID NO: 6, or a fragment thereof) at a level significantly above background. The invention also includes use of the poly-nucleotides that encode the lysyl oxidase described herein, as well as nucleotide sequences which are complementary thereto. The nucleotide sequence may be RNA or DNA, including genomic DNA, synthetic DNA or cDNA. Preferably, the nucleotide sequence is DNA and most preferably, a synthetic DNA sequence. Typically, a polynucleotide used in the invention comprises a contiguous sequence of nucleotides which is capable of hybridizing under selective conditions to the coding sequence or the complement of the coding sequence of SEQ_ID NO:6. Such nucleotides can be synthesized according to methods well known in the art.

A polynucleotide used in the invention can hybridize to the coding sequence or the complement of the coding sequence of SEQ_ID NO: 6 at a level significantly above background. Background hybridization may occur, for example, because of other cDNAs present in a cDNA library. The signal level generated by the interaction between a polynucleotide used in the invention and the coding sequence or complement of the coding sequence of SEQ_ID NO: 6 is typically at least 10 fold, preferably at least 20 fold, more preferably at least 50 fold, and even more preferably at least 100 fold, as intense as interactions between other polynucleotides and the coding sequence of SEQ_ID NO: 6. The intensity of interaction may be measured, for example, by radiolabelling the probe, for example with ³²P. Selective hybridization may typically be achieved using conditions of low stringency (0.3M sodium chloride and 0.03M sodium citrate at about 40°C), medium stringency (for example, 0.3M sodium chloride and 0.03M sodium citrate at about 50°C) or high stringency (for example, 0.3M sodium chloride and 0.03M sodium citrate at about 60°C).

A poly-nucleotide used in the invention also includes synthetic genes that can encode for the poly-peptide of SEQ_ID NO: 1, SEQ_ID NO: 2, SEQ_ID NO: 3 or SEQ_ID NO: 4 or variants thereof. It is sometimes preferable to adapt the codon usage of a gene to the preferred bias in a production host. Techniques to design and construct synthetic genes are generally available (i.e http://www.dnatwopointo.com/).

### Modifications

Poly-nucleotides usd in the invention may comprise DNA or RNA. They may be single or double stranded. They may also be poly-nucleotides which include within them synthetic or modified nucleotides including peptide nucleic acids. A number of different types of modifications to poly-nucleotides are known in the art. These include a methylphosphonate and phosphorothioate backbones, and addition of acridine or polylysine chains at the 3' and/or 5' ends of the molecule. For the purposes of the present invention, it is to be understood that the poly-nucleotides described herein may be modified by any method available in the art.

It is to be understood that skilled persons may, using routine techniques, make nucleotide substitutions that do not affect the polypeptide sequence encoded by the poly-nucleotides used in the invention to reflect the codon usage of any particular host organism in which the polypeptides of the invention are to be expressed.

The coding sequence of SEQ_ID NO: 6 may be modified by nucleotide substitutions, for example from 1, 2 or 3 to 10, 25, 50, 100, or more substitutions. The polynucleotide of SEQ_ID NO: 6 may alternatively or additionally be modified by one or more insertions and/or deletions and/or by an extension at either or both ends. The modified polynucleotide generally encodes a polypeptide which has lysyl oxidase activity. Degenerate substitutions may be made and/or substitutions may be made which would result in a conservative amino acid substitution when the modified sequence is translated, for example as discussed with reference to polypeptides later.

### Homologues

A nucleotide sequence which is capable of selectively hybridizing to the complement of the DNA coding sequence of SEQ_ID NO: 6 is included in the invention and will generally have at least 50% or 60%, at least 70%, at least 80%, at least 90%, at least 95%, at least 98% or at least 99% sequence identity to the coding sequence of SEQ_ID NO: 6 over a region of at least 60, preferably at least 100, more preferably at least 200 contiguous nucleotides or most preferably over the full length of SEQ_ID NO:6. Likewise, a nucleotide which encodes an active lysyl oxidase and which is capable of selectively hybridizing to a fragment of a complement of the DNA coding sequence of SEQ_ID NO: 6, is also embraced by the invention. Any combination of the above mentioned degrees of identity and minimum sizes may be used to define poly-nucleotides suitable for use in the invention, with the more stringent combinations (i.e. higher identity over longer lengths) being preferred. Thus, for example, a polynucleotide which is at least 80% or 90% identical over 60, preferably over 100 nucleotides, may be used in the invention, as may a polynucleotide which is at least 90% identical over 200 nucleotides.

The BLASTP and BLAST N algorithms can be used to calculate sequence identity or to line up sequences (such as identifying equivalent or corresponding sequences, for example on their default settings).

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighborhood word score threshold. These initial neighborhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program from DNA-DNA comparison uses as defaults a word length (W) of 11, expectation (E) of 10, and a comparison of both strands. The BLASTP program for protein-protein comparison uses as defaults a word length (W) of 3, the BLOSUM62 scoring matrix, a gap existence penalty of 11 with an gap extension penalty of 1, and an expectation (E) of 10.

The BLAST algorithm performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

### Primers and Probes

Poly-nucleotides as described herein include and may be used as primers, for example as polymerase chain reaction (PCR) primers, as primers for alternative amplification reactions, or as probes for example labeled with a revealing label by conventional means using radioactive or non-radioactive labels, or the poly-nucleotides may be cloned into vectors. Such primers, probes and other fragments will be at least 15, for example at least 20, 25, 30 or 40 nucleotides in length. They will typically be up to 40, 50, 60, 70, 100, 150, 200 or 300 nucleotides in length, or even up to a few nucleotides (such as 5 or 10 nucleotides) short of the coding sequence of SEQ_ID NO:6.

In general, primers will be produced by synthetic means, involving a step-wise manufacture of the desired nucleic acid sequence one nucleotide at a time. Techniques for accomplishing this and protocols are readily available in the art. Longer polynucleotides will generally be produced using recombinant means, for example using PCR cloning techniques. This will involve making a pair of primers (typically of about 15-30 nucleotides) to amplify the desired region of the lysyl oxidase to be cloned, bringing the primers into contact with mRNA, cDNA or genomic DNA obtained from a yeast, bacterial, plant, prokaryotic or fungal cell, preferably of an *Aspergillus* strain, performing a polymerase chain reaction under conditions suitable for the amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Alternatively, synthetic genes can be constructed that encompass the coding region of the secreted lysyl oxidase or variants thereof. Poly-nucleotides that are altered in many positions, but still encode the same protein can be conveniently be designed and constructed using these techniques. This has as advantage that the codon usage can be adapted to the preferred expression host, so productivity of the protein in this host can be improved. Also the polynucleotide sequence of a gene can be changed to improve mRNA stability or reduced turnover. This can lead to improved expression of the desired protein or variants thereof. Additionally, the poly-nucleotide sequence can be changed in a synthetic gene such that mutations are made in the protein sequence that have a positive effect on secretion efficiency, stability, proteolytic vulnerability, temperature optimum, specific activity or other relevant properties for industrial production or application of the protein. Companies that provide services to construct synthetic genes and optimize codon usage are generally available.

Such techniques may be used to obtain all or part of the poly-nucleotides encoding the lysyl oxidase sequences described herein. Introns, promoter and trailer regions are within the scope of the invention and may also be obtained in an analogous manner (e.g. by recombinant means, PCR or cloning techniques), starting with genomic DNA from a fungal, yeast, bacterial plant or prokaryotic cell.

The poly-nucleotides or primers may carry a revealing label. Suitable labels include radioisotopes such as ³²P or ³⁵S, fluorescent labels, enzyme labels, or other protein labels such as biotin. Such labels may be added to poly-nucleotides or primers for use in the invention and may be detected using techniques known to persons skilled in the art.

Poly-nucleotides or primers (or fragments thereof) labeled or unlabelled may be used in nucleic acid-based tests for detecting or sequencing a lysyl oxidase or a variant thereof in a fungal sample. Such detection tests will generally comprise bringing a fungal sample suspected of containing the DNA of interest into contact with a probe comprising a polynucleotide or primer described herein under hybridizing conditions, and detecting any duplex formed between the probe and nucleic acid in the sample. Detection may be achieved using techniques such as PCR or by immobilizing the probe on a solid support, removing any nucleic acid in the sample which is not hybridized to the probe, and then detecting any nucleic acid which is hybridized to the probe. Alternatively, the sample nucleic acid may be immobilized on a solid support, the probe hybridized and the amount of probe bound to such a support after the removal of any unbound probe detected.

The probes for use in the invention may conveniently be packaged in the form of a test kit in a suitable container. In such kits the probe may be bound to a solid support where the assay format for which the kit is designed requires such binding. The kit may also contain suitable reagents for treating the sample to be probed, hybridizing the probe to nucleic acid in the sample, control reagents, instructions, and the like. The probes and poly-nucleotides may also be used in micro-assay.

Preferably, the polynucleotide for use in the invention is obtainable from the same organism as the polypeptide, such as a fungus, in particular a fungus of the genus *Aspergillus.*

### Production of poly-nucleotides

Poly-nucleotides which do not have 100% identity with SEQ_ID NO: 6 but which may be used in the invention can be obtained in a number of ways. Thus, variants of the lysyl oxidase sequence described herein may be obtained for example, by probing genomic DNA libraries made from a range of organisms, such as those discussed as sources of the polypeptides for use in the invention. In addition, other fungal, plant or prokaryotic homologues of lysyl oxidase may be obtained and such homologues and fragments thereof in general will be capable of hybridizing to SEQ_ID NO: 6. Such sequences may be obtained by probing cDNA libraries or genomic DNA libraries from other species, and probing such libraries with probes comprising all or part of SEQ_ID NO: 6 under conditions of low, medium to high stringency (as described earlier). Nucleic acid probes comprising all or part of SEQ_ID NO: 6 may be used to probe cDNA or genomic libraries from other species, such as those described as sources for the polypeptides for use in the invention.

Species homologues may also be obtained using degenerate PCR, which uses primers designed to target sequences within the variants and homologues which encode conserved amino acid sequences. The primers can contain one or more degenerate positions and will be used at stringency conditions lower than those used for cloning sequences with single sequence primers against known sequences. A preferable way to obtain species homologues of lysyl oxidase is to design primers that target sequences that encode the consensus sequences described in SEQ_ID NO: 11.

Alternatively, such poly-nucleotides may be obtained by site directed mutagenesis of the lysyl oxidase sequences or variants thereof. This may be useful where, for example, silent codon changes to sequences are required to optimize codon preferences for a particular host cell in which the polynucleotide sequences are being expressed. Other sequence changes may be made in order to introduce restriction enzyme recognition sites, or to alter the property or function of the polypeptides encoded by the poly-nucleotides.

Double stranded poly-nucleotides comprising a polynucleotide described herein and its complement may be use in the invention.

Herein is described poly-nucleotides encoding the polypeptides for use in the invention described above. Since such poly-nucleotides will be useful as sequences for recombinant production of polypeptides for use in the invention, it is not necessary for them to be capable of hybridizing to the sequence of SEQ_ID NO: 6, although this will generally be desirable. Otherwise, such poly-nucleotides may be labeled, used, and made as described above if desired.

### Recombinant Poly-nucleotides.

Described herein are vectors comprising a polynucleotide for use in the invention, including cloning and expression vectors, and in another aspect methods of growing, transforming or transfecting such vectors into a suitable host cell, for example under conditions in which expression of a polypeptide for, or encoded by a sequence for, use in the invention occurs. Provided also are host cells comprising a polynucleotide or vector described herein wherein the polynucleotide is heterologous to the genome of the host cell. The term "heterologous", usually with respect to the host cell, means that the polynucleotide does not naturally occur in the genome of the host cell or that the polypeptide is not naturally produced by that cell. Preferably, the host cell is a yeast cell, for example a yeast cell of the genus *Kluyveromyces, Pichia, Hansenula* or *Saccharomyces* or a filamentous fungal cell, for example of the genus *Aspergillus, Trichoderma* or *Fusarium.*

### Vectors

The vector into which the expression cassette described herein is inserted may be any vector that may conveniently be subjected to recombinant DNA procedures, and the choice of the vector will often depend on the host cell into which it is to be introduced. Thus, the vector may be an autonomously replicating vector, i.e. a vector which exists as an extra-chromosomal entity, the replication of which is independent of chromosomal replication, such as a plasmid. Alternatively, the vector may be one which, when introduced into a host cell, is integrated into the host cell genome and replicates together with the chromosome(s) into which it has been integrated.

Preferably, when a polynucleotide as described herein is in a vector it is operably linked to a regulatory sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence such as a promoter, enhancer or other expression regulation signal "operably linked" to a coding sequence is positioned in such a way that expression of the coding sequence is achieved under production conditions.

The vectors may, for example in the case of plasmid, cosmid, virus or phage vectors, be provided with an origin of replication, optionally a promoter for the expression of the polynucleotide and optionally an enhancer and/or a regulator of the promoter. A terminator sequence may be present, as may be a poly-adenylation sequence. The vectors may contain one or more selectable marker genes, for example an ampicillin resistance gene in the case of a bacterial plasmid or a neomycin resistance gene for a mammalian vector. Vectors may be used *in vitro,* for example for the production of RNA or can be used to transfect or transform a host cell.

The DNA sequence encoding the polypeptide is preferably introduced into a suitable host as part of an expression construct in which the DNA sequence is operably linked to expression signals which are capable of directing expression of the DNA sequence in the host cells. For transformation of the suitable host with the expression construct transformation procedures are available which are well known to the skilled person. The expression construct can be used for transformation of the host as part of a vector carrying a selectable marker, or the expression construct is co-transformed as a separate molecule together with the vector carrying a selectable marker. The vectors may contain one or more selectable marker genes.

Preferred selectable markers include but are not limited to those that complement a defect in the host cell or confer resistance to a drug. They include for example versatile marker genes that can be used for transformation of most filamentous fungi and yeasts such as acetamidase genes or cDNAs (the amdS, niaD, facA genes or cDNAs from *A.nidulans, A.oryzae,* or *A.niger*)*,* or genes providing resistance to antibiotics like G418, hygromycin, bleomycin, kanamycin, phleomycin or benomyl resistance (benA). Alternatively, specific selection markers can be used such as auxotrophic markers which require corresponding mutant host strains: e.g. URA3 (from *S.cerevisiae* or analogous genes from other yeasts), pyrG or pyrA (from *A.nidulans* or *A.niger*), argB (from *A.nidulans* or *A.niger*) or trpC. In a preferred embodiment the selection marker is deleted from the transformed host cell after introduction of the expression construct so as to obtain transformed host cells capable of producing the polypeptide which are free of selection marker genes.

Other markers include ATP synthetase subunit 9 (oliC), orotidine-5'-phosphate-decarboxylase (pvrA), the bacterial G418 resistance gene (useful in yeast, but not in filamentous fungi), the ampicillin resistance gene (*E*. *coli*), the neomycin resistance gene (*Bacillus*) and the *E*. *coli* uidA gene, coding for glucuronidase (GUS). Vectors may be used *in vitro,* for example for the production of RNA or to transfect or transform a host cell.

For most filamentous fungi and yeast, the expression construct is preferably integrated into the genome of the host cell in order to obtain stable transformants. However, for certain yeasts suitable episomal vector systems are also available into which the expression construct can be incorporated for stable and high level expression. Examples thereof include vectors derived from the 2 µm, CEN and pKD1 plasmids of *Saccharomyces* and *Kluyveromyces,* respectively, or vectors containing an AMA sequence (e.g. AMA1 from *Aspergillus*). When expression constructs are integrated into host cell genomes, the constructs are either integrated at random loci in the genome, or at predetermined target loci using homologous recombination, in which case the target loci preferably comprise a highly expressed gene. A highly expressed gene is a gene whose mRNA can make up at least 0.01 % (w/w) of the total cellular mRNA, for example under induced conditions, or alternatively, a gene whose gene product can make up at least 0.2% (w/w) of the total cellular protein, or, in case of a secreted gene product, can be secreted to a level of at least 0.05 g/l.

An expression construct for a given host cell will usually contain the following elements operably linked to each other in consecutive order from the 5'-end to 3'-end relative to the coding strand of the sequence encoding the polypeptide of the first aspect: (1) a promoter sequence capable of directing transcription of the DNA sequence encoding the polypeptide in the given host cell, (2) preferably, a 5'-untranslated region (leader), (3) optionally, a signal sequence capable of directing secretion of the polypeptide from the given host cell into the culture medium, (4) the DNA sequence encoding a mature and preferably active form of the polypeptide, and preferably also (5) a transcription termination region (terminator) capable of terminating transcription downstream of the DNA sequence encoding the polypeptide.

Downstream of the DNA sequence encoding the polypeptide, the expression construct preferably contains a 3' untranslated region containing one or more transcription termination sites, also referred to as a terminator. The origin of the terminator is less critical. The terminator can for example be native to the DNA sequence encoding the polypeptide. However, preferably a bacterial terminator is used in bacterial host cells, a yeast terminator is used in yeast host cells and a filamentous fungal terminator is used in filamentous fungal host cells. More preferably, the terminator is endogenous to the host cell in which the DNA sequence encoding the polypeptide is expressed.

Enhanced expression of the polynucleotide encoding the polypeptide for use in the invention may also be achieved by the selection of heterologous regulatory regions, e.g. promoter, signal sequence and terminator regions, which serve to increase expression and, if desired, secretion levels of the protein of interest from the chosen expression host and/or to provide for the inducible control of the expression of the polypeptide for use in the invention.

Aside from the promoter native to the gene encoding the polypeptide for use in the invention, other promoters may be used to direct expression of the polypeptide for use in the invention. The promoter may be selected for its efficiency in directing the expression of the polypeptide for use in the invention in the desired expression host.

Promoters/enhancers and other expression regulation signals may be selected to be compatible with the host cell for which the expression vector is designed. For example prokaryotic promoters may be used, in particular those suitable for use in *E.coli* strains. When expression of the polypeptides for use in the invention is carried out in mammalian cells, mammalian promoters may be used. Tissues-specific promoters, for example hepatocyte cell-specific promoters, may also be used. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, herpes simplex virus promoters or adenovirus promoters.

Suitable yeast promoters include the S. *cerevisiae* GAL4 and ADH promoters and the S. *pombe* nmt1 and adh promoter. Mammalian promoters include the metallothionein promoter which can be induced in response to heavy metals such as cadmium. Viral promoters such as the SV40 large T antigen promoter or adenovirus promoters may also be used. All these promoters are readily available in the art.

Mammalian promoters, such as ß-actin promoters, may be used. Tissue-specific promoters, in particular endothelial or neuronal cell specific promoters (for example the DDAHI and DDAHII promoters), are especially preferred. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, adenovirus, HSV promoters (such as the HSV IE promoters), or HPV promoters, particularly the HPV upstream regulatory region (URR). Viral promoters are readily available in the art.

A variety of promoters can be used that are capable of directing transcription in the host cells as described herein. Preferably the promoter sequence is derived from a highly expressed gene as previously defined. Examples of preferred highly expressed genes from which promoters are preferably derived and/or which are comprised in preferred predetermined target loci for integration of expression constructs, include but are not limited to genes encoding glycolytic enzymes such as triose-phosphate isomerases (TPI), glyceraldehyde-phosphate dehydrogenases (GAPDH), phosphoglycerate kinases (PGK), pyruvate kinases (PYK), alcohol dehydrogenases (ADH), as well as genes encoding amylases, glucoamylases, proteases, xylanases, cellobiohydrolases, ß-galactosidases, alcohol (methanol) oxidases, elongation factors and ribosomal proteins. Specific examples of suitable highly expressed genes include e.g. the LAC4 gene from *Kluyveromyces* sp., the methanol oxidase genes (AOX and MOX) from *Hansenula* and *Pichia,* respectively, the glucoamylase (glaA) genes from *A.niger* and *A.awamori,* the *A.oryzae* TAKA-amylase gene, the *A.nidulans* gpdA gene and the *T.reesei* cellobiohydrolase genes.

Examples of strong constitutive and/or inducible promoters which are preferred for use in fungal expression hosts are those which are obtainable from the fungal genes for xylanase (xlnA), phytase, ATP-synthetase subunit 9 (oliC), triose phosphate isomerase (tpi), alcohol dehydrogenase (AdhA), amylase (amy), amyloglucosidase (AG - from the glaA gene), acetamidase (amdS) and glyceraldehyde-3-phosphate dehydrogenase (gpd) promoters.

Examples of strong yeast promoters which may be used include those obtainable from the genes for alcohol dehydrogenase, glyceraldehyde-3-phosphate dehydrogenase, lactase, 3-phosphoglycerate kinase, plasma membrane ATPase (PMA1) and triosephosphate isomerase.

Examples of strong bacterial promoters which may be used include the amylase and SPo2 promoters as well as promoters from extracellular protease genes.

Promoters suitable for plant cells which may be used include napaline synthase (nos), octopine synthase (ocs), mannopine synthase (mas), ribulose small subunit (rubisco ssu), histone, rice actin, phaseolin, cauliflower mosaic virus (CMV) 35S and 19S and circovirus promoters.

The vector may further include sequences flanking the polynucleotide giving rise to RNA which comprise sequences homologous to ones from eukaryotic genomic sequences, preferably fungal genomic sequences, or yeast genomic sequences. This will allow the introduction of the polynucleotides as described herein into the genome of fungi or yeasts by homologous recombination. In particular, a plasmid vector comprising the expression cassette flanked by fungal sequences can be used to prepare a vector suitable for delivering the polynucleotides described herein to a fungal cell. Transformation techniques using these fungal vectors are known to those skilled in the art.

The vector may contain a polynucleotide as described herein oriented in an antisense direction to provide for the production of antisense RNA. This may be used to reduce, if desirable, the levels of expression of the polypeptide.

### Host Cells and Expression

In a further aspect the invention provides a process for preparing a polypeptide for use in the invention which comprises cultivating a host cell transformed or transfected with an expression vector as described above under conditions suitable for expression by the vector of a coding sequence encoding the polypeptide, and recovering the expressed polypeptide. Polynucleotides as described herein can be incorporated into a recombinant replicable vector, such as an expression vector. The vector may be used to replicate the nucleic acid in a compatible host cell. Herein is also desribed a method of making a polynucleotide by introducing a polynucleotide as described herein into a replicable vector, introducing the vector into a compatible host cell, and growing the host cell under conditions which bring about the replication of the vector. Suitable host cells include bacteria such as *E*. *coli,* yeast, mammalian cell lines and other eukaryotic cell lines, for example insect cells such as Sf9 cells and (e.g. filamentous) fungal cells.

Preferably the polypeptide is produced as a secreted protein in which case the DNA sequence encoding a mature form of the polypeptide in the expression construct may be operably linked to a DNA sequence encoding a signal sequence. In the case where the gene encoding the secreted protein has in the wild type strain a signal sequence preferably the signal sequence used will be native (homologous) to the DNA sequence encoding the polypeptide. Alternatively the signal sequence is foreign (heterologous) to the DNA sequence encoding the polypeptide, in which case the signal sequence is preferably endogenous to the host cell in which the DNA sequence is expressed. Examples of suitable signal sequences for yeast host cells are the signal sequences derived from yeast MFalpha genes. Similarly, a suitable signal sequence for filamentous fungal host cells is e.g. a signal sequence derived from a filamentous fungal amyloglucosidase (AG) gene, e.g. the *A.niger* glaA gene. This signal sequence may be used in combination with the amyloglucosidase (also called (gluco) amylase) promoter itself, as well as in combination with other promoters. Hybrid signal sequences may also be used within the context of the present invention.

Preferred heterologous secretion leader sequences are those originating from the fungal amyloglucosidase (AG) gene (glaA - both 18 and 24 amino acid versions e.g. from *Aspergillus*), the MFalpha gene (yeasts e.g. *Saccharomyces* and *Kluyveromyces*) or the alpha-amylase gene (*Bacillus*).

The vectors may be transformed or transfected into a suitable host cell as described above to provide for expression of a polypeptide for use in the invention. This process may comprise culturing a host cell transformed with an expression vector as described above under conditions suitable for expression of the polypeptide, and optionally recovering the expressed polypeptide.

Also described herein are host cells transformed or transfected with or comprising a polynucleotide or vector for use in the invention. Preferably the polynucleotide is carried in a vector which allows the replication and expression of the polynucleotide. The cells will be chosen to be compatible with the said vector and may for example be prokaryotic (for example bacterial), or eukaryotic fungal, yeast or plant cells.

The invention encompasses processes for the production of a polypeptide for use in the invention by means of recombinant expression of a DNA sequence encoding the polypeptide. For this purpose the DNA sequence described herein can be used for gene amplification and/or exchange of expression signals, such as promoters, secretion signal sequences, in order to allow economic production of the polypeptide in a suitable homologous or heterologous host cell. A homologous host cell is herein defined as a host cell which is of the same species or which is a variant within the same species as the species from which the DNA sequence is derived.

Suitable host cells are preferably prokaryotic microorganisms such as bacteria, or more preferably eukaryotic organisms, for example fungi, such as yeasts or filamentous fungi, or plant cells. In general, yeast cells are preferred over filamentous fungal cells because they are easier to manipulate. However, some proteins are either poorly secreted from yeasts, or in some cases are not processed properly (e.g. hyperglycosylation in yeast). In these instances, a filamentous fungal host organism should be selected.

Bacteria from the genus *Bacillus* are very suitable as heterologous hosts because of their capability to secrete proteins into the culture medium. Other bacteria suitable as hosts are those from the genera *Streptomyces* and *Pseudomonas.* A preferred yeast host cell for the expression of the DNA sequence encoding the polypeptide is one of the genus *Saccharomyces, Kluyveromyces, Hansenula, Pichia, Yarrowia,* or *Schizosaccharomyces.* More preferably, a yeast host cell is selected from the group consisting of the species *Saccharomyces cerevisiae, Kluyveromyces lactis* (also known as *Kluyveromyces marxianus var. lactis), Hansenula polymorpha, Pichia pastoris, Yarrowia lipolytica,and Schizosaccharomyces pombe.*

Most preferred for the expression of the DNA sequence encoding the polypeptide are, however, filamentous fungal host cells. Preferred filamentous fungal host cells are selected from the group consisting of the genera *Aspergillus, Trichoderma, Fusarium, Disporotrichum, Penicillium, Acremonium, Neurospora, Thermoascus, Myceliophtora, Sporotrichum, Thielavia,* and *Talaromyces.* More preferably a filamentous fungal host cell is of the species *Aspergillus oyzae, Aspergillus sojae* or *Aspergillus nidulans* or is of a species from the *Aspergillus niger* Group (as defined by Raper and Fennell, The Genus Aspergillus, The Williams & Wilkins Company, Baltimore, pp 293-344, 1965). These include but are not limited to *Aspergillus niger, Aspergillus awamori, Aspergillus tubigensis, Aspergillus aculeatus, Aspergillus foetidus, Aspergillus nidulans, Aspergillus japonicus, Aspergillus oryzae* and *Aspergillus ficuum,* and also those of the species *Trichoderma reesei, Fusarium graminearum, Penicillium chrysogenum, Acremonium alabamense, Neurospora crassa, Myceliophtora thermophilum, Sporotrichum cellulophilum, Disporotrichum dimorphosporum* and *Thielavia terrestris.*

Examples of preferred expression hosts are fungi such as *Aspergillus* species (in particular those described in EP-A-184,438 and EP-A-284,603) and *Trichoderma* species; bacteria such as *Bacillus* species (in particular those described in EP-A-134,048 and EP-A-253,455), especially *Bacillus subtilis, Bacillus licheniformis, Bacillus amyloliquefaciens, Pseudomonas* species; and yeasts such as *Kluyveromyces* species (in particular those described in EP-A-096,430 such as *Kluyveromyces lactis* and in EP-A-301,670) and *Saccharomyces* species, such as *Saccharomyces cerevisiae.*

Host cells include plant cells, and therefore extend to transgenic organisms, such as plants and parts thereof, which contain one or more cells as described herein. The cells may heterologously express the polypeptide for use in the invention or may heterologously contain one or more of the polynucleotides as described herein. The transgenic (or genetically modified) plant may therefore have inserted (typically stably) into its genome a sequence encoding the polypeptides for use in the invention. The transformation of plant cells can be performed using known techniques, for example using a Ti or a Ri plasmid from *Agrobacterium tumefaciens.* The plasmid (or vector) may thus contain sequences necessary to infect a plant, and derivatives of the Ti and/or Ri plasmids may be employed.

The host cell may overexpress the polypeptide, and techniques for engineering over-expression are well known.. The host may thus have two or more copies of the polynucleotide.

Alternatively, direct infection of a part of a plant, such as a leaf, root or stem can be effected. In this technique the plant to be infected can be wounded, for example by cutting the plant with a razor, puncturing the plant with a needle or rubbing the plant with an abrasive. The wound is then innoculated with the *Agrobacterium.* The plant or plant part can then be grown on a suitable culture medium and allowed to develop into a mature plant. Regeneration of transformed cells into genetically modified plants can be achieved by using known techniques, for example by selecting transformed shoots using an antibiotic and by sub-culturing the shoots on a medium containing the appropriate nutrients, plant hormones and the like.

### Culture of host cells and recombinant production

Described herein are cells that have been modified to express the lysyl oxidase or a variant thereof. Such cells include transient, or preferably stably modified higher eukaryotic cell lines, such as mammalian cells or insect cells, lower eukaryotic cells, such as yeast and filamentous fungal cells or prokaryotic cells such as bacterial cells.

It is also possible for the polypeptides for use in the invention to be transiently expressed in a cell line or on a membrane, such as for example in a baculovirus expression system. Such systems, which are adapted to express the proteins for use in the invention, are also described herein.

According to the present invention, the production of the polypeptide for use in the invention can be effected by the culturing of microbial expression hosts, which have been transformed with one or more polynucleotides as described herein, in a conventional nutrient fermentation medium.

The recombinant host cells may be cultured using procedures known in the art. For each combination of a promoter and a host cell, culture conditions are available which are conducive to the expression the DNA sequence encoding the polypeptide. After reaching the desired cell density or titre of the polypeptide the culturing is ceased and the polypeptide is recovered using known procedures.

The fermentation medium can comprise a known culture medium containing a carbon source (e.g. glucose, maltose, molasses, etc.), a nitrogen source (e.g. ammonium sulphate, ammonium nitrate, ammonium chloride, etc.), an organic nitrogen source (e.g. yeast extract, malt extract, peptone, etc.) and inorganic nutrient sources (e.g. phosphate, magnesium, potassium, zinc, iron, etc.). Optionally, an inducer (dependent on the expression construct used) may be included or subsequently be added.

The selection of the appropriate medium may be based on the choice of expression host and/or based on the regulatory requirements of the expression construct. Suitable media are well-known to those skilled in the art. The medium may, if desired, contain additional components favoring the transformed expression hosts over other potentially contaminating microorganisms.

The fermentation may be performed over a period of from 0.5-30 days. Fermentation may be a batch, continuous or fed-batch process, at a suitable temperature in the range of between 0°C and 45°C and, for example, at a pH from 2 to 10. Preferred fermentation conditions include a temperature in the range of between 20°C and 37°C and/or a pH between 3 and 9. The appropriate conditions are usually selected based on the choice of the expression host and the protein to be expressed.

After fermentation, if necessary, the cells can be removed from the fermentation broth by means of centrifugation or filtration. After fermentation has stopped or after removal of the cells, the polypeptide of the invention may then be recovered and, if desired, purified and isolated by conventional means. The lysyl oxidase for use in the invention can be purified from fungal mycelium or from the culture broth into which the lysyl oxidase is released by the cultured fungal cells.

In a preferred embodiment the polypeptide produced from a fungus, more preferably from an *Aspergillus,* most preferably from *Aspergillus niger.*

### Modifications

Polypeptides for use in the invention may be chemically modified, e.g. post-translationally modified. For example, they may be glycosylated (one or more times) or comprise modified amino acid residues. They may also be modified by the addition of histidine residues to assist their purification or by the addition of a signal sequence to promote secretion from the cell. The polypeptide may have amino- or carboxyl-terminal extensions, such as an amino-terminal methionine residue, a small linker peptide of up to about 20-25 residues, or a small extension that facilitates purification, such as a poly-histidine tract, an antigenic epitope or a binding domain.

A polypeptide for use in the invention may be labelled with a revealing label. The revealing label may be any suitable label which allows the polypeptide to be detected. Suitable labels include radioisotopes, e.g. ¹²⁵I, ³⁵S, enzymes, antibodies, polynucleotides and linkers such as biotin.

The polypeptides may be modified to include non-naturally occurring amino acids or to increase the stability of the polypeptide. When the proteins or peptides are produced by synthetic means, such amino acids may be introduced during production. The proteins or peptides may also be modified following either synthetic or recombinant production.

The polypeptides for use in the invention may also be produced using D-amino acids. In such cases the amino acids will be linked in reverse sequence in the C to N orientation. This is conventional in the art for producing such proteins or peptides.

A number of side chain modifications are known in the art and may be made to the side chains of the proteins or peptides for use in the present invention. Such modifications include, for example, modifications of amino acids by reductive alkylation by reaction with an aldehyde followed by reduction with NaBH₄, amidination with methylacetimidate or acylation with acetic anhydride.

The sequences described herein may also be used as starting materials for the construction of "second generation" enzymes. "Second generation" lysyl oxidases are lysyl oxidases, altered by mutagenesis techniques (e.g. site-directed mutagenesis or gene shuffling techniques), which have properties that differ from those of wild-type lysyl oxidase or recombinant lysyl oxidase such as those described herein. For example, their temperature or pH optimum, specific activity, substrate affinity or thermostability may be altered so as to be better suited for use in a particular process.

Amino acids essential to the activity of the lysyl oxidase described herein, and therefore preferably subject to substitution, may be identified according to procedures known in the art, such as site-directed mutagenesis or alanine-scanning mutagenesis. In the latter technique mutations are introduced at every residue in the molecule, and the resultant mutant molecules are tested for biological activity (e.g. lysyl oxidase activity) to identify amino acid residues that are critical to the activity of the molecule. Sites of enzyme-substrate interaction can also be determined by analysis of crystal structure as determined by such techniques as nuclear magnetic resonance, crystallography or photo-affinity labelling.

Gene shuffling techniques provide a random way to introduce mutations in a polynucleotide sequence. After expression the isolates with the best properties are re-isolated, combined and shuffled again to increase the genetic diversity. By repeating this procedure a number of times, genes that code for fastly improved proteins can be isolated. Preferably the gene shuffling procedure is started with a family of genes that code for proteins with a similar function. The family of polynucleotide sequences described herein would be well suited for gene shuffling to improve the properties of secreted lysyl oxidases.

Alternatively classical random mutagenesis techniques and selection, such as mutagenesis with NTG treatment or UV mutagenesis, can be used to improve the properties of a protein. Mutagenesis can be performed directly on isolated DNA, or on cells transformed with the DNA of interest. Alternatively, mutations can be introduced in isolated DNA by a number of techniques that are known to the person skilled in the art. Examples of these methods are error-prone PCR, amplification of plasmid DNA in a repear-deficient host cell, etc.

The use of yeast and filamentous fungal host cells is expected to provide for post-translational modifications (e.g. proteolytic processing, myristilation, glycosylation, truncation, and tyrosine, serine or threonine phosphorylation) as may be needed to confer optimal biological activity on recombinant expression products for use in the invention.

### Preparations

Polypeptides for use in the invention may be in an isolated form. It will be understood that the polypeptide may be mixed with carriers or diluents which will not interfere with the intended purpose of the polypeptide and still be regarded as isolated. A polypeptide for use in the invention may also be in a substantially purified form, in which case it will generally comprise the polypeptide in a preparation in which more than 70%, e.g. more than 80%, 90%, 95%, 98% or 99% of the proteins in the preparation is a polypeptide as described herein.

Polypeptides for use in the invention may be provided in a form such that they are outside their natural cellular environment. Thus, they may be substantially isolated or purified, as discussed above, or in a cell in which they do not occur in nature, for example a cell of other fungal species, animals, plants or bacteria.

### Removal or reduction of lysyl oxidase activity

Herein is described methods for producing a mutant cell of a parent cell, which comprises disrupting or deleting the endogenous nucleic acid sequence encoding the polypeptide or a control sequence thereof, which results in the mutant cell producing less of the polypeptide than the parent cell.

The construction of strains which have reduced lysyl oxidase activity may be conveniently accomplished by modification or inactivation of a nucleic acid sequence necessary for expression of the lysyl oxidase in the cell. The nucleic acid sequence to be modified or inactivated may be, for example, a nucleic acid sequence encoding the polypeptide or a part thereof essential for exhibiting lysyl oxidase activity, or the nucleic acid sequence may have a regulatory function required for the expression of the polypeptide from the coding sequence of the nucleic acid sequence. An example of such a regulatory or control sequence may be a promoter sequence or a functional part thereof, i.e., a part which is sufficient for affecting expression of the polypeptide. Other control sequences for possible modification include, but are not limited to, a leader sequence, a poly-adenylation sequence, a pro-peptide sequence, a signal sequence, and a termination sequence.

Modification or inactivation of the nucleic acid sequence may be performed by subjecting the cell to mutagenesis and selecting cells in which the lysyl oxidase producing capability has been reduced or eliminated. The mutagenesis, which may be specific or random, may be performed, for example, by use of a suitable physical or chemical mutagenizing agent, by use of a suitable oligo-nucleotide, or by subjecting the DNA sequence to PCR mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (NTG), O-methyl hydroxylamine, nitrous acid, ethyl methane sulphonate (EMS), sodium bisulphite, formic acid, and nucleotide analogues.

When such agents are used, the mutagenesis is typically performed by incubating the cell to be mutagenized in the presence of the mutagenizing agent of choice under suitable conditions, and selecting for cells exhibiting reduced or no expression of lysyl oxidase activity.

Modification or inactivation of production of a polypeptide suitable for use in the present invention may be accomplished by introduction, substitution, or removal of one or more nucleotides in the nucleic acid sequence encoding the polypeptide or a regulatory element required for the transcription or translation thereof. For example, nucleotides may be inserted or removed so as to result in the introduction of a stop codon, the removal of the start codon, or a change of the open reading frame. Such modification or inactivation may be accomplished by site-directed mutagenesis or PCR mutagenesis in accordance with methods known in the art.

Although, in principle, the modification may be performed *in vivo,* i.e., directly on the cell expressing the nucleic acid sequence to be modified, it is preferred that the modification be performed *in vitro* as exemplified below.

An example of a convenient way to inactivate or reduce production of the lysyl oxidase by a host cell of choice is based on techniques of gene replacement or gene interruption. For example, in the gene interruption method, a nucleic acid sequence corresponding to the endogenous gene or gene fragment of interest is mutagenized in vitro to produce a defective nucleic acid sequence which is then transformed into the host cell to produce a defective gene. By homologous recombination, the defective nucleic acid sequence replaces the endogenous gene or gene fragment. Preferably the defective gene or gene fragment also encodes a marker which may be used to select for transformants in which the gene encoding the polypeptide has been modified or destroyed.

Alternatively, modification or inactivation of the nucleic acid sequence encoding a polypeptide for use in the present invention may be achieved by established anti-sense techniques using a nucleotide sequence complementary to the polypeptide encoding sequence. More specifically, production of the polypeptide by a cell may be reduced or eliminated by introducing a nucleotide sequence complementary to the nucleic acid sequence encoding the polypeptide. The anti-sense poly-nucleotide will then typically be transcribed in the cell and will be capable of hybridizing to the mRNA encoding the lysyl oxidase. Under conditions allowing the complementary anti-sense nucleotide sequence to hybridize to the mRNA, the amount of the lysyl oxidase produced in the cell will be reduced or eliminated.

It is preferred that the cell to be modified in accordance with the methods of the present invention is of microbial origin, for example, a fungal strain which is suitable for the production of desired protein products, either homologous or heterologous to the cell.

Herein is further described a mutant cell of a parent cell which comprises a disruption or deletion of the endogenous nucleic acid sequence encoding the polypeptide or a control sequence thereof, which results in the mutant cell producing less of the polypeptide than the parent cell.

The polypeptide-deficient mutant cells so created are particularly useful as host cells for the expression of homologous and/or heterologous polypeptides. Therefore, there are methods described herein for producing a homologous or heterologous polypeptide comprising (a) culturing the mutant cell under conditions conducive for production of the polypeptide; and (b) recovering the polypeptide. In the present context, the term "heterologous polypeptides" is defined herein as polypeptides which are not native to the host cell, a native protein in which modifications have been made to alter the native sequence, or a native protein whose expression is quantitatively altered as a result of a manipulation of the host cell by recombinant DNA techniques.

Also described herein is a method for producing a protein product essentially free of lysyl oxidase activity by fermentation of a cell which produces both a lysyl oxidase poly-peptide for use in the present invention as well as the protein product of interest. The method comprises adding an effective amount of an agent capable of inhibiting lysyl oxidase activity to the fermentation broth either during or after the fermentation has been completed, recovering the product of interest from the fermentation broth, and optionally subjecting the recovered product to further purification. Alternatively, after cultivation the resultant culture broth can be subjected to a pH or temperature treatment so as to reduce the lysyl oxidase activity substantially, and allow recovery of the product from the culture broth. The combined pH or temperature treatment may be performed on a protein preparation recovered from the culture broth.

The methods described herein for producing an essentially lysyl oxidase-free product is of particular interest in the production of eukaryotic polypeptides, in particular in the production of fungal proteins such as enzymes. The lysyl oxidase-deficient cells may also be used to express heterologous proteins of interest for the food industry, or of pharmaceutical interest.

### Peptide motifs to identify genes

A peptide motif can be used to identify genes that code for proteins containing this peptide motif. Instead of one peptide motif, also a combination of two or more peptide motifs can be used to identify genes coding for proteins containing the peptide motifs. When one or several peptide motifs coding for specific lysyl oxidases are identified it is thus possible to identify genes coding for lysyl oxidases using one or a combination of several of these peptide motifs. Lysyl oxidases are used as an example how such genes may be identified, but the methods described are generally applicable. A possibility is to use the sequence of the motif of SEQ_ID NO: 11 for a search in translated DNA sequences from a DNA databank or protein sequences from a protein sequence databank using a program like Patscan (http://www-unix.mcs.anl.gov/compbio/PatScan/HTML/). The amino acid sequence has to be entered in a special format that is described on the website. Another method that can be performed is to to use the sequence of the motif of SEQ_ID NO: 11 for a search in translated DNA sequences from a DNA databank or protein sequences from a protein sequence databank using a program like http://myhits.isb-sib.ch/cgi-bin/. For this program the motif is entered in the search field in the so called Prosite format, and databases are searched for the presence of the motif in the protein sequence or in the translated DNA sequence. This method is further described in Example 1 of this invention, using the amino acid motif of SEQ_ID NO: 11, and is used to identify fungal genes that encode useful lysyl oxidases. The genes that are identified using one of these methods can than be translated into a protein sequence using programs known to those skilled in the art, and be inspected for the presence of a signal sequence at their amino-terminus. For detecting a signal sequence one can use a program like SignalP (http://www.cbs.dtu.dk/services/SignalP/). In the current invention we have found that a protein sequence that contains both the consensus sequences and a predicted signal sequence is likely to be a secreted lysyl oxidase. Looking for these combined properties gives a large advantage for the industrial production of such an enzyme.

Another possibility to identify lysyl oxidase genes using peptide motifs is to design oligo-nucleotide primers based on the back-translation of the amino acid sequence of the motif of SEQ_ID NO: 11 into a nucleotide sequence with preferred codon usage from the organism in which one wants to identify a lysyl oxidase gene, and using this oligo-nucleotide for hybridization to a gene library, or in a PCR primer on a reverse transcribed mRNA pool. Using peptide sequence motif SEQ_ID NO: 11, it is also possible to isolate genes encoding lysyl oxidase when the gene sequence is unknown. Methods have been described in literature to design degenerate oligo-nucleotide primers that can be used for this purpose (Sambrook et al. (1989) Molecular cloning: a laboratory manual. Cold Spring Harbor Laboratory Press). Also, methods to isolate genes from an organism, using a degenerate oligo-nucleotide as probe or primer, have been described.

Oligo-nucleotides that code for the peptide motif of SEQ_ID NO: 11 are useful for isolation of the genes encoding lysyl oxidase properties. The degeneracy of such a group of oligo-nucleotides may be decreased by the introduction of inosine (I) bases at positions where the nucleotide is not known. Additionally, positions where both cytosine (C) and thimidine (T) bases are possible may be replaced by uracil (U), and at positions where both adenine (A) and guanine (G) are possible only guanine may be introduced, in order to decrease degeneracy with only a small effect on specificity of the oligo-nucleotide primer. Furthermore, for screening the presence of genes encoding lysyl oxidases in organisms of which the codon preference is known, the degeneracy of the oligo-nucleotide can be further decreased by taking the codon preference into account in the design of the oligo-nucleotide. A person skilled in the art will know how to do this. Furthermore, all possible combinations of oligo-nucleotide primers, without degeneracy, may be synthesized separately and used in individual screening experiments.

First, a genomic, cDNA or EST library is constructed from the species of interest in a universal vector. Suitable methods for library construction are described in literature (Sambrook et al. (1989) Molecular cloning: a laboratory manual. Cold Spring Harbor Laboratory Press). Second, a degenerate oligo-nucleotide described above is used in a PCR reaction together with one universal oligo-nucleotide that primes in the vector, at the border of the recombinant DNA insert, on DNA isolated from the library. Useful strategies have been described in literature for the isolation of a desired gene when only a single degenerate oligo-nucleotide primer is available (e.g. Minambres et al. (2000) Biochem. Biophys. Res. Commun. 272, 477-479; PCR technology (1989) Ed. H.A. Erlich pp. 99-104, Stockton Press). Third, the PCR amplified fragment is then labeled and used as probe for the screening of the library by conventional means. The full length gene can than be sub-cloned into an expression vector suitable for over-expression of the lysyl oxidase in a desired production host organism.

In a different approach, when no library is available from the species that is screened for the presence of a gene encoding a lysyl oxidase, part of the gene can be amplified by PCR with different degenerate primers, or with 3'-RACE using a single degenerate primer. For this, RNA is isolated from the species of interest and used in a 3'-RACE reaction using a single degenerate primer as gene specific primer. The amplification of part of an unknown cDNA using one degenerate oligo-nucleotide and one universal primer, by 3'-RACE, has been described previously (WO99/38956).

The traditional method to isolate a full-length gene using the information from only a small peptide is hybridization of a labelled degenerate oligo-nucleotide to filters on which a library is replicated. Methods describing the screening of gene libraries using degenerate oligo-nucleotides, and methods to calculate or determine the optimal hybridization conditions of these oligo-nucleotides, have been extensively described in literature (Sambrook et al.(1989)). The oligo-nucleotides described above may be used for this method to isolate genes encoding a lysyl oxidase from different species.

In a variation to this method, a partial gene library can be constructed first. For this, DNA is fractionated, after which fragments of DNA containing the gene coding for a lysyl oxidase are detected by hybridization to the labelled oligo-nucleotides described above. These fragments are isolated and used in the construction of a partial gene library enriched in the gene coding for a lysyl oxidase. This library can than be screened by conventional means. For this method, genomic DNA is first digested with restriction enzymes before fractionation by gel-electrophoresis, while cDNA can be fractionated directly.

A different method to isolate the gene coding for a lysyl oxidase is by using antibodies raised against any of the peptides of the consensus sequence. Antibodies may be monoclonal or polyclonal. Methods describing the production of antibodies specific for small peptides have been extensively described in literature (Harlow, E and Lane, D (1988) Antibodies; a laboratory manual, ISBN 0-87969-314. -2).

Expression libraries can be constructed from the species of interest, by cloning cDNA or genomic DNA into a vector suitable for expressing the insert in a convenient host, such as *E*. *coli* or yeast. Expression vectors may or may not be based on phage lambda. Immuno-detection of antigens produced by expression libraries, and methods describing the purification of specific clones expressing the antigen have been published. Using an antibody specific for any of the peptides of the consensus motif, it is possible to isolate the gene encoding a lysyl oxidase encompassing this motif, using this method.

In effect, many different methods may be used to isolate a gene coding for a lysyl oxidase when the information described in this invention is taken into account. The advantage of using the peptide motif sequence information over prior art methods is the speed and relative ease with which a new gene coding for an active lysyl oxidase can be identified. The use of the sequence information gives an indication of the value of a new lysyl oxidase in cheese making, or other applications in food industry, without performing laborious testing of all possible oxidases in direct application experiments.

We will demonstrate the invention in the examples below. We will demonstrate the identification, cloning, production, purification and biochemical characterization of the novel lysyl oxidases. We will further demonstrate the use of the lysyl oxidases in the preparation of food stuffs.

### Legends to the figures

Figure 1: Construction of lysyl oxidase expression vectors pGBFINZGR-1, pGBFINZGT-1, pGBFINGLO-1, pGBFINZGY-1 and pGBFINGLO-2. The gene of interest is labeled as Xxx in this Figure which code can be replaced by any of the codes of Table 1
Figure 2: SDS-PAGE profiles showing protein expression of lysyl oxidases in *Aspergillus niger.* Samples are taken from culture supernatants and spotted on SDS-PAGE (10% NUPAGE, Invitrogen). C: control, host strain without transformed plasmid. M: marker proteins (116, 66, 45, 35, 25, 18 and 14 kDa), ZGR, ZGT, GLO-1, GLO-2 and ZGY: overexpressed lysyl oxidases. Arrows indicate location of overexpressed protein.

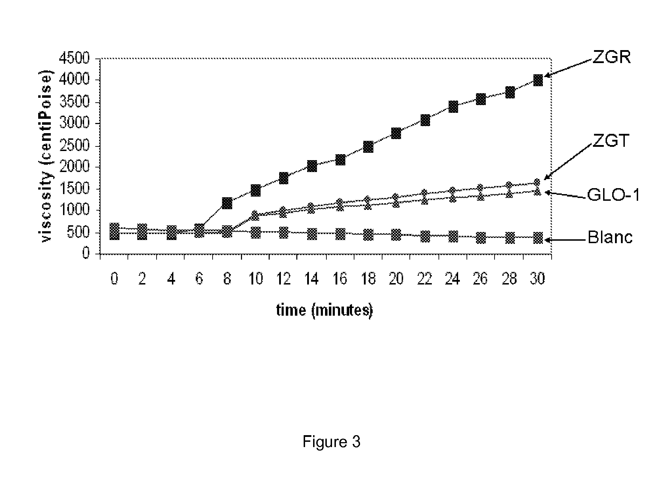

Figure 3: Viscosity development of a 12.5 % whey solution at 85°C, with or without treatment with lysyl oxidases ZGR, ZGT or GLO-1.
Figure 4. SDS-PAGE profiles of κ-casein, α-casein and a mixture of κ- and α-casein and the same profiles after incubation with lysyl oxidase ZGR

### Sequences

SEQ_ID NO 1: Aspergillus nidulans ZGR protein
SEQ_ID NO 2: Cryptococcus neoformans ZGT protein
SEQ_ID NO 3: Fusarium graminearum GLO1 protein
SEQ_ID NO 4: Podospora anserina ZGY protein
SEQ_ID NO 5: Fusarium graminearum GLO2 protein
SEQ_ID NO 6: Aspergillus nidulans ZGR synthetic DNA
SEQ_ID NO 7: Cryptococcus neoformans ZGT synthetic DNA
SEQ_ID NO 8: Fusarium graminearum GLO1 genomic DNA
SEQ_ID NO 9: Podospora anserina ZGY synthetic DNA
SEQ_ID NO 10: Fusarium graminearum GLO2 genomic DNA
SEQ_ID NO 11: consensus motif: IHD[NS]LSGSMHDHV[IL]NFK

### EXAMPLES

### Example 1

### Identification of putative lysyl oxidase encoding genes in a fungal genome sequence

Secreted lysyl oxidases from fungal origin are interesting to produce in large amounts since they may be used in food applications. Currently, the only described secreted lysyl oxidase from a filamentous fungus is from *Aspergillus oryzae,* and is described in SEQ_ID NO: 2 of EP 1466979 A1. Here we have attempted to overexpress this *Aspergillus oryzae* enzyme in the fungus *Aspergillus niger.* Surprisingly, no lysyl oxidase activity could be measured in the culture fluid after overexpression of this sequence in *Aspergillus niger.* To explain this surprising result, we have examined the amino acid sequence described in EP 1466979 A1 in more detail. Surprisingly, the sequence of the putative *Aspergillus oryzae* lysyl oxidase is in some aspects different on important positions from the sequence of other lysyl oxidases and copper amine oxidases. I.e position 164 of the annotated protein of the *Asperillus oryzae* gene described in SEQ_ID NO: 2 of EP 1466979 A1 is an alanine, while in all other described amine and lysyl oxidases this position is highly conserved to proline. Additionally position 78 in this annotated protein is a methionine, while leucine is highly conserved in amine oxidases at this position. Furthermore, the protein described in EP 1466979 A1 contains an extra insertion of 2 amino acids after residue 109 when compared to mammalian and *Pichia* lysyl oxidase. Also position 499 in the the protein described in EP 1466979 A1 is a threonine, while this position is always a hydrophobic residue in other amine oxidases, and residue. Conserved positions in proteins are often associated with the primairy function of an enzyme, and cannot be changed without changing the catalytic activity of the enzyme. It is therefore doubtful if the protein sequence described in EP 1466979 A1 is indeed correct and codes for a functional lysyl oxidase. The oxidase activity that has been measured in some of the transformants in EP 1466979 A1 might therefore not be due to the overexpression of the described gene.

Since the approach to overexpress the gene from EP 1466979 A1 was unsuccessfull, we carefully examined the amino acid sequence of lysyl oxidases, and propose here a amino acid sequence motif that is usefull in the identification of lysyl oxidases from fungi. The motif that we propose to be useful to identify genuine fungal lysyl oxidases is:
I-H-D-[NS]-L-S-G-S-M-H-D-H-V-[IL]-N-F-K (SEQ_ID NO:11)

In this motif every amino acid is represented as standard IUPAC one-letter codes, and where more amino acids are possible at a single position, the possible amino acids are in brackets.

We searched trEMBL (translated EMBL DNA database) with motif SEQ_ID NO:11 with the pattern search option at http://myhits.isb-sib.ch/cgi-bin/. This program can search translated DNA or protein databases with a defined motif such as SEQ_ID NO:11. For this the motif is entered in the pattern-search field in the so called Prosite format, such as depicted above, and individual databases are searched for the presence of the motif in the protein sequence or in the translated DNA sequence. Using this method we were able to identify 6 potential lysyl oxidases from fungal origin that had not been assigned to this activity before and all contained the motif SEQ_ID NO:11. The genes that code for 4 of the 6 new potential lysyl oxidases are mentioned in Table 1, and originate from *Aspergillus nidulans, Cryptococcus neoformans, Fusarium graminearum* and *Podospora anserina.* Besides these 4 sequences, also a second gene from *Cryptococcus neoformans* (Q5KEB2_CRYNE) and a gene from *Neurospora crassa* (Q7S286_NEUCR) can be found with motif SEQ_ID NO:11. The 6 sequences are all from fungi from phylogenetically widely separated species, being from the two separate phyla Basidiomycota and Ascomycota, suggesting that the protein motifs are highly conserved in evolution and code for biochemically relevant amino acid residues. Interestingly, all deduced protein sequences contain a putative signal sequence at the amino-terminus of the protein, and might therefore code for secreted proteins.

To test if the sequence motifs are indeed useful for the identification of lysyl oxidases with relevant activity, we have cloned and overexpressed the genes coding for the 4 new proteins mentioned in Table 1. As a control, we also included a second gene from *Fusarium graminearum* with clear homology to all 4 other lysyl oxidases, but lacking the amino acid sequence motif. Using this control experiment we can investigate if the use of the motif is indeed useful for the identification and detection of new relevant lysyl oxidases from fungal origin.

**Table 1: List with properties of expressed enzymes**

| **Donor organism** | **Plasmid name** | **Protein** | **Code** | **Motif** | **Expression** | **Accession (trEMBL)** |
|---|---|---|---|---|---|---|
| *Aspergillus nidulans* | pGBFINZGR-1 | SEQ_ID NO 1 | ZGR | + | +++ | Q5B038_E MENI |
| *Cryptococcus neoformans* | pGBFINZGT-1 | SEQ_ID NO 2 | ZGT | + | + | Q55P44_C RYNE |
| *Fusarium graminearum* | pGBFINGLO-1 | SEQ_ID NO 3 | GLO1 | + | +++ | Q4HWI1_GI BZE |
| *Podospora anserina* | pGBFINZGY-1 | SEQ_ID NO 4 | ZGY | + | ++ | Q86ZN4_P ODAN |
| *Fusarium graminearum* | pGBFINGLO-2 | SEQ_ID NO 5 | GLO2 | - | +++ | Q4HWS1_G IBZE |

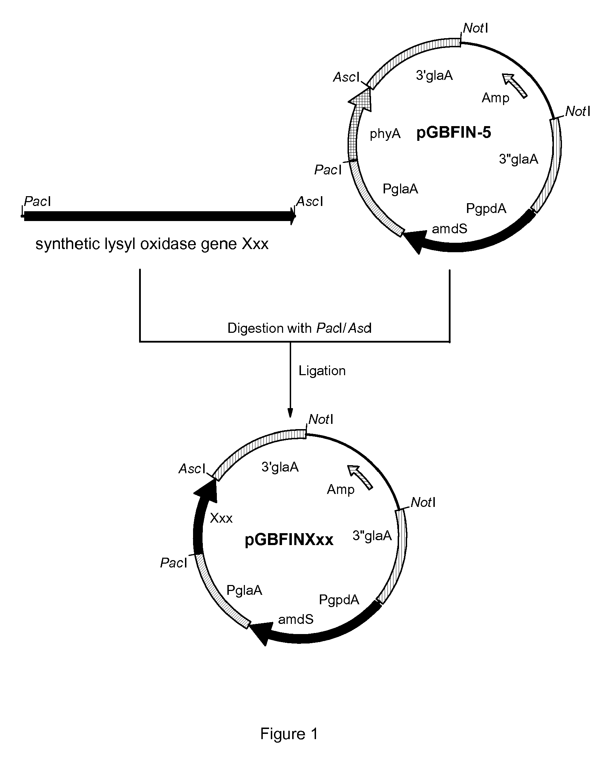

Synthetic genes that can encode the proteins described above were made by DNA2.0 (http://www.dnatwopointo.com/). It is possible to synthetically produce any gene sequence and use it in cloning and expression. The synthetic genes (SEQ_ID NO:6, 7, and 9) were designed using an algorithm that adapts the coding sequence to the codon bias for highly expressed genes in *Aspergillus niger* (WO 06/077258). The exception to this was the sequence of the two *Fusarium* genes where the genomic DNA sequence was taken (SEQ_ID NO:8 and 10). The used gene sequences code for the proteins mentioned in respectively SEQ_ID NO: 1-5. Cloning sites *PacI* and *AscI* were introduced upstream respectively downstream of the coding sequences of these enzymes, in order to facilitate the cloning procedure. The fragments containing the synthetic putative lysyl oxidase genes were digested with *PacI* and *AscI,* and the *PacI*/*Asc*I fragment comprising the coding sequences was isolated and exchanged with the *Pac*I/*Asc*I *phy*A fragment in pGBFIN-5 (WO 99/32617) using standard molecular biology methods. The resulting expression plasmids were used for the expression of the different genes in *Aspergillus niger,* and are named as described in Table 1. The cloning procedure and the structure of the expression plasmids is depicted in Figure 1. In place of the synthetic gene Xxx any of the codes of the genes mentioned in Table 1 can be filled in, resulting in the plasmids mentioned in Table 1. The coding sequence of the genes of interest are cloned downstream from the *glaA* promoter of *Aspergillus niger,* and the *amdS* gene of *Aspergillus nidulans* is present on the expression plasmids as marker for selection in *Aspergillus niger.*

### Example 2

### Transformation and over expression of putative fungal lysyl oxidases by A. niger

The expression vectors pGBFINZGR-1, pGBFINZGT-1, pGBFINGLO-1, pGBFINZGY-1, and pGBFINGLO-2 were linearized by digestion with *Not*I, which removes all *E*. *coli* derived sequences from the expression vector. The digested DNA was purified using phenol:chloroform:isoamylalcohol (24:23:1) extraction and precipitation with ethanol. These vectors were used to transform *Aspergillus niger* CBS513.88. An *Aspergillus niger* transformation procedure is extensively described in WO 98/46772. It is also described how to select for transformants on agar plates containing acetamide, and to select targeted multicopy integrants. Preferably, *A. niger* transformants containing multiple copies of the expression cassette are selected for further generation of sample material. For the lysyl oxidase expression vectors 30 *A.* nigertransformants were purified for every transformed plasmid; first by plating individual transformants on selective medium plates followed by plating a single colony on PDA plates. Spores of individual transformants were collected after growth for 1 week at 30 degrees Celsius. Spores were stored refrigerated and were used for the inoculation of liquid media.

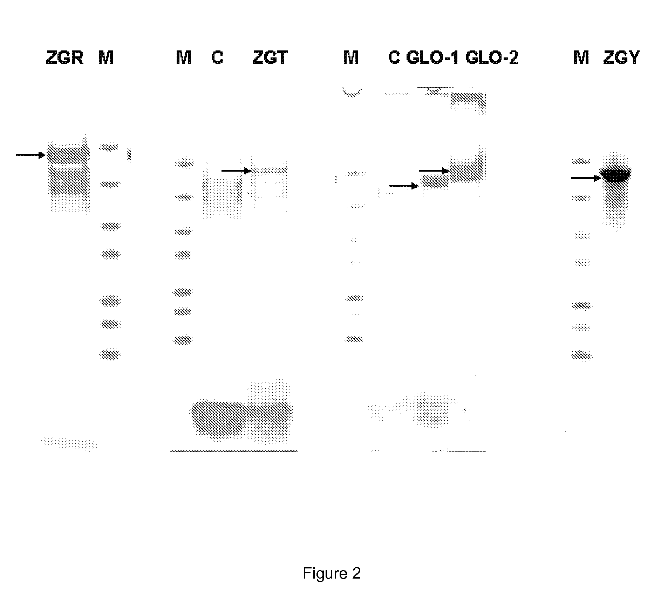

An *A. niger* strain containing multiple copies of the expression cassette was used for generation of sample material by cultivation of the strain in shake flask cultures. A useful method for cultivation of *A. niger* strains and separation of the mycelium from the culture broth is described in WO 98/46772. Cultivation medium was in CSM-MES (150 g maltose, 60 g Soytone (Difco), 15 g (NH₄)₂SO₄, 1 g NaH₂PO₄·H₂O, 1 g MgSO₄·7H₂O, 1 g L-arginine, 80 mg Tween-80, 20 g MES pH6.2 per liter medium). 5 ml samples were taken on day 4-8 of the fermentation, centrifuged for 10 min at 5000 rpm in a Hereaus labofuge RF and supernatants were stored at -20°C until further analyses. Figure 2 shows protein expression profiles, as determined by SDS-PAGE (Coomassie-staining) of the supernatants after cultivation. Clearly, the cloned lysyl-oxidases from *Aspergillus nidulans, Cryptococcus neoformans, Fusarium graminearum* and *Podospora anserina* show excellent overexpession, showing they are well expressed and excreted. This is highly surprising and unexpected since the *Aspergillus oryzae* enzyme, which is much closer related to the host *Aspergillus niger,* could not be over-expressed although it had been reported previously (EP1 466 979) that this annotated *Aspergillus oryzae* lysyl oxidase could be expressed in a heterologous host. The four lysyl oxidase genes, of which all donor-organisms are less related to *Aspergillus niger,* were expressed efficiently in this host. Also the gene GLO2 from *Fusarium graminearum* was efficiently expressed and secreted from *Aspergillus niger.*

### Example 3

### Purification of the over expressed lysyl oxidase from an A. niger supernatant

Lysyl oxidases derived from *Aspergillus nidulans, Cryptococcus neoformans, Fusarium graminearum* and *Podospora anserina* that were over expressed in *Aspergillus niger* were cultivated in larger quantities as follows. Transformed *A. niger* strains expressing the relevant lysyl oxidase were grown in a growth medium containing per liter maltose.H₂O: 40 g; Soytone (Difco): 30 g; (NH₄)₂SO₄: 15 g, NaH₂PO₄·H₂O: 1g; MgSO₄·7H₂O: 1, L-arginine: 1 g, Tween-80: 0.08 g, Na-citrate: 70 g. The pH was adjusted to 6.2. After 5-6 days of growth at 30 degrees C, cells were killed off by adding 3.5 g/l of sodium benzoate and prolonging incubation for another 6 hours. Then 10 g/l of CaCl2 and 45 g/l filter aid (Dicalite BF; Gent, Belgium) were added to the broth. Mycelium was removed by an initial cloth filtration, followed by filtration through Z-2000 and Z-200 filters (Pall). Ultrafiltration was carried out using an Amicon ultrafiltration cell with a 10 kDa membrane until a 2-4 fold volume reduction. The purification of the lysyl oxidases was performed as follows:

### Lysyl oxidase ZGR (derived from Aspergillus nidulans

The concentrated culture supernatant containing lysyl oxidase ZGR was equilibrated in 20 mM Tris.HCl (pH7.5; buffer A1) to a conductivity of 1.8 mS/cm in the Amicon ultra filtration cell. The sample was subsequently loaded on a Q-Sepharose column (5 ml Q-FF Hitrap, Pharmacia) using a flow rate of 5 ml/min. After washing with 3 column volumes (cv) of buffer A1 the lysyl oxidase was eluted using a 20 cv linear gradient from buffer A1 to B1 (B1 = buffer A1 + 1M NaCl). Lysyl oxidase containing fractions, eluting around 340 mM NaCl, were identified via SDS-PAGE, pooled and transferred to an Amicon concentration cell with a 10 kDa membrane to change buffer to 25 mM Na-phosphate (pH6.6; buffer A2). The sample was than applied to a Q-Sepharose column (5 ml Q-FF Hitrap, Pharmacia, 5 ml/min), equilibrated in buffer A2. After washing with 3 cv buffer A2 lysyl oxidase was eluted in a 20 cv linear gradient from buffer A2 to buffer B2 (B2 = buffer A2 + 1 M NaCl). Lysyl oxidase containing fractions were pooled. The enzyme was at least 95% pure, as determined by SDS-PAGE (Coomassie staining).

### Lysyl oxidase ZGT (derived from Cryptococcus neoformans)

Lysyl oxidase ZGT was purified essentially as described for ZGR with the following adaptations: buffers A1 and B1 were at pH7.1 instead of pH7.5. The lysyl oxidase eluted in the first step at 330 mM NaCl, in the second column it eluted at 470 mM NaCl. The protein was at least 90% pure, as determined by SDS-PAGE (Coomassie staining).

### Lysyl oxidase GLO-1 (derived from Fusarium graminearum).

Lysyl oxidase GLO-1 was purified essentially as described for ZGR with the following adaptations: buffers A1 and B1 were at pH7.0 instead of pH7.5; buffer A2 contained 20 mM Tris.HCl (pH7.7) and buffer B2 contained 20 mM Tris.HCl (pH7.7) + 1 M NaCl. GLO-1 eluted in the first purification step at 90 mM NaCl, in the second purification step it was not bound to the column and was present in the flow through. The protein was at least 95% pure, as determined by SDS-PAGE (Coomassie staining).

### Lysyl oxidase GLO-2 (derived from Fusarium graminearum)

Lysyl oxidase GLO-2 was purified essentially as dwescribed for ZGR with the following adaptations: buffers A1 and B1 were at pH7.0 instead of pH7.5; buffer A2 contained 20 mm Tris.HCl (pH7.9) and buffer B2 contained 20 mM Tris.HCl (pH7.9) + 1 M NaCl. GLO-2 eluted the first purification step in two peaks at respectively 80 and 200 mM, indicating some protein heterogeneity. This was not further examined. Both peaks were pooled and used in the second purification step, in which the enzyme eluted again in two fractions at 180 and 400 mM respectively, with the majority of the protein (∼80%) eluting at 180 mM. This fraction was pooled and used for further analysis.

### Lysyl oxidase ZGY (derived from Podospora anserina)

Lysyl oxidase ZGY was purified essentially as described for ZGR with the following adaptations. Buffer A1 contained 50 mM Na-acetate (pH5.0) and buffer B1 contained 50 mM Na-acetate (pH5.0) + 1M NaCl. In the second purification step, a hydroxyapatite column was used (XK16/20, 30 ml, Pharmacia, 2 ml/min). Buffer A2 contained 10 mM Na-phosphate (pH6.8) and buffer B2 contained 500 mM Na-phosphate (pH6.8). The protein was at least 95% pure, as determined by SDS-PAGE (Coomassie-staining).
Protein concentrations were determined using the Bradford reagent.

### Example 4

### Activity determination of lysyl oxidases

The enzymatic activity of lysyl oxidase on an amine substrate results in the formation of H₂O₂ and NH₃. Both reaction products can be used to monitor enzyme activity.

### Lysyl oxidase activity monitoring the formation of H₂O₂.

Lysyl oxidase activity was monitored using the Amplex Red Mono-amine oxidase assay Kit, obtainable from Invitrogen. The reactions were carried out according to the manufacturers instructions. Substrate concentration in the assay solution were 1 mM, unless indicated otherwise. Reactions were performed at 30°C unless indicated otherwise, and the reaction was followed in time using a Genios microtiterplate reader (TECAN). For the determination of the pH optimum of the enzyme the following buffers were used: 25 mM Na-citrate (pH3.0, pH6.0), 25 mM Na-acetate (pH4.0, pH5.0), 25 mM Tris.HCl (pH7.0, pH8.0) and Tris.Glycine (pH9.0, pH10.0).

### Lysyl oxidase activity monitoring the formation of NH₃

Lysyl oxidase activity was determined by incubating the enzyme (100 microgram enzyme per ml assay solution) for 4 hours at 37°C under gentle shaking (500 rpm) in open Eppendorff tubes, in a solution containing 10 mM Ac-Gly-Lys-OMe (obtained from Bachem, Germany) and 25 mM Na-phosphate buffer (pH 7.0) Formation of NH₃ was detected with ammonia test strips (obtained from Hach, Germany). In a blanc experiment water was added instead of enzyme solution. The formation of a dark green colour signals the presence of NH_{3;} absence of ammonia leaves a yellow color.

The lysyl oxidases ZGR, ZGT, GLO-1, GLO-2 and ZGY were tested for lysyloxidase activity in the Amplex Red assay using the following substrates (10.0 mM in assay solution): Ac-Gly-Lys-OMe, Ac-Lys-OMe (both obtained from Bachem, Germany), benzylamine and p-tyramine (Invitrogen). In case of the lysine-containing substrates the only free amino group is the one in the lysine side chain since the alpha-amino group is blocked. Any activity on these substrates therefore unequivocally signals lysyl-oxidase activity. The results are given below; activity is presented as % of the activity obtained with the substrate showing maximum activity. The data for the *Pichia* enzyme were calculated from the k_{cat}/Kₘ values published Kuchar & Dooley (J Inorg Biochem (2001) 83, 193-204)

**Table 1: Activity of lysyl oxidases on several substrates. The activity of the enzyme on benzylamine is set at 100%; the other activities are expressed as % of this activity.**

| Enzyme code | Ac-Gly-Lys-OMe | Ac-Lys-OMe | p-Tyramine | Benzylamine |
|---|---|---|---|---|
| ZGR | 182 | 157 | 122 | 100 |
| ZGT | 208 | 162 | 174 | 100 |
| GLO-1 | 204 | 167 | 96 | 100 |
| GLO-2 | 32 | 87 | 213 | 100 |
| ZGY | 385 | 262 | 239 | 100 |
| *Pichia pastoris* | 95 | Not determined | Not determined | 100 |

The enzymes all show clear preference for Ac-Gly-Lys-OMe as expected for lysyl oxidases. It is clear that ZGR, ZGT, GLO-1 and ZGY have a surprisingly high preference for the lysyl group, and the activity is higher when the peptide substrate is larger (Ac-Gly-Lys-OMe vs Ac-LysOMe). Surprisingly, their preference for lysine substrates is even stronger than that of the *Pichia* lysyl oxidase. Where the *Pichia* derived lysyl oxidase is even a little more active on benzyl amine compared to Ac-Gly-Lys-OMe, the ZGR, ZGT, GLO-1 and ZGY have a clear preference for the lysyl-substrate. Also the GLO-2 protein has a clear preference, like the Pichia enzyme, for small substrates like benzylamine and tyramine. Surprisingly, the four lysyl oxidases that were identified using the amino acid sequence motifs had higher preference for the lysine substrates, and lysyl oxidases that lack one of the motifs have a less preferred activity spectrum. Apparently the search with the amino acid motifs identifies specifically the lysyl oxidases with more preferred activity on lysine substrates, and is therefore an important new tool in the identification of useful lysyl oxidases. It should be noted that the lysyl oxidases that are found using the motif SEQ_ID NO:11 are only 40-60% identical to each other using BLASTP, but apparently all have in common that the specificity of these enzymes is more towards a higher preference for lysine substrates. Enzymes with similar homology scores using BLASTP but lacking the motif SEQ_ID NO:11, like GLO2 but also the lysyl oxidases from *Pichia pastoris* and *Aspergillus oryzae,* do not show this substrate specificity.

The enzymes ZGR, ZGT and GLO-1 were also tested on ammonia release, using Ac-Gly-Lys-OMe as the substrate. In all cases the release of ammonia was unequivocally demonstrated. These results further confirm that the identified enzymes are lysyl oxidases.

### Example 5

### Activity of lysyl oxidases on various protein substrates

Lysyl oxidases ZGR, ZGT and GLO-1 were tested for activity at pH6 on the following protein substates: whey (BiPro form Davisco), casein (obtained from DMV, The Netherlands;), gluten hydrolysate (vital gluten (obtained from Protinax) was hydrolyzed with Fromase 750TL (obtained from DSM, The Netherlands) for 1 hour at 55°C, pH4.1. The enzyme was inactivated by heat treatment at 85°C, 15 minutes and spray dried). For reference purposes, Ac-Gly-Lys-OMe was used under conditions as described before. The results are given below. The activity of the various lysyl oxidases is qualified as form ++++ (very active, comparable to Ac-Gly-Lys-OMe), +++ (well active) ++ (moderately active), + little active, - not active.

**Table 2: Activity of ZGR, ZGT and GLO-1 on whey, casein and gluten proteins.**

| Enzyme | Whey | Casein | Gluten | Ac-Gly-Lys-OMe |
|---|---|---|---|---|
| ZGR | + | ++++ | ++++ | ++++ |
| ZGT | + | ++++ | ++ | ++++ |
| GLO-1 | - | +/- | + | ++++ |
| GLO-2 | - | - | - | + |

Clearly, the lysyl oxidases differ in their activity among each other, and also in their activity towards various proteins. Whey proteins are most difficult to modify by the lysyl oxidases, whereas casein is the best accessible substrate for ZGR and ZGT. ZGR is also very active on gluten, where ZGT is only moderately active. GLO-1 is the least active of the three lysyl oxidases, showing no detectable activity on whey proteins, almost no activity on casein and little activity on gluten protein. Nevertheless, the results clearly demonstrate that the enzymes are active on lysine residues in proteins, as expected for lysyl oxidases. As expected based on the results of example 4, GLO-2 showed no activity on any of the protein substrates.

### Example 6

### Determination of pH and T profiles of lysyl oxidases

The pH profiles of lysyl oxidases were determined over the pH-range 3-8, using buffers as indicated in example 4. The assay was performed in two steps. First the reaction was carried out at the proper pH during a fixed period of time. Then the solution was diluted in the standard buffer for the Amplex Red assay (H₂O₂) and the initial rate was determined. The recording of the pH profile was performed at 30°C using benzylamine as the substrate. The results are given in table 3.

**Table 3. Optimum pH and pH range in which lysyl oxidases show >80% of maximum activity.**

| Enzyme | pH optimum | pH range with >80% of maximum activity | T-optimum (°C) |
|---|---|---|---|
| ZGR | 4.0 | 3.5-7.0 | 30 |
| ZGT | 5.0 | 4.5-5.5 | <20 |
| GLO-1 | 6.0 | 5.5-6.5 | 50 |
| ZGY | 9.0 | 8.0-10.0 | 40 |

The table shows that the lysyl oxidases have different pH optima, ranging from pH4 to pH9. The lysyl oxidase with the broadest pH range is ZGR (3.5 pH units). The temperature-optima of these enzymes were determined at their pH optimum and are given in table 3. The GLO-1 enzyme shows the highest T-optimum (50°C). The ZGR, ZGT and ZGY have relatively low T-optima, but in temperature ranges well compatible with many food applications. The T-optimum of ZGT is remarkably low, even below 20°C.

### Example 7

### Demonstration of a quinone structure in lysyl oxidases

Lysyl oxidases are known to contain a quinone structure in their active site. We therefore verified the presence of such cofactor in the identified lysyl oxidases using the following procedure. Lysyl oxidases ZGR, ZGT and GLO-1 were applied on a SDS-PAGE (10% bis-Tris NuPage, Invitrogen) followed by Western blotting onto nitrocellulose paper. Quinones were visualized by staining the blot with NBT (nitro-tetrazolium blue chloride) (0.24 mM in 2M Na-glycinate, pH10). The presence of a quinone structure results in the appearance of purple color on the place where the protein is located. ZGR, ZGT and GLO-1 clearly showed the formation of this purple color. As control, an endo-protease (Maxiren600, obtained form DSM, The Netherlands) and a carboxy-peptidase (Accelerzyme CPG, obtained from DSM, The Netherlands) were included. These enzymes are known to have no quinone group, and did not show a purple color upon staining with NBT. This experiment shows that the lysyl-oxidases ZGR, ZGT and GLO-1 contain a quinone structure, as expected for lysyl oxidases. The exact nature of the quinone can not be derived from this experiment.

### Example 8

### Use of lysyl oxidases to modify the texture of concentrated whey solutions upon heating

In the first step of the process, WPI (BiPro, Davisco) was dissolved in MilliQ water to 10.5% and incubated overnight at room temperature with ZGR, ZGT or GLO-1 (10 mg enzyme / 25 ml solution). The treatment with the lysyl oxidase did not result in any significant change in viscosity of the protein solution, indicating absence or low levels (<1%) of protein cross linking. In a control experiment, water was added instead of enzyme solution. The solutions were then transferred to a measuring cell of a Rapid Visco Analyzer (RVA-4 NewPort Scientific, Australia), equilibrated at 85°C. In this second step of the process, the reactive intermediates, formed during the first step of the process are allowed to react under formation of covalent bonds. The solution was slowly stirred (50 rpm) and the change in viscosity was followed. Figure 5 clearly shows that in the blanc, no change in viscosity occurs indicating no gel formation. In contrast, addition of the lysyl oxidases results in a significant increase in viscosity. This is especially pronounced for ZGR. Gel formation clearly occurs as a result of the treatment with the lysyl oxidase, evidencing the reactivity of the reactive protein substates prepared in the first step of the process Surprisingly, even GLO-1 has effect even though in example 4 no activity of this lysyl oxidase on whey protein was observed. The reaction in example 4 was monitored over only a few minutes, whereas in the current example the enzyme was allowed to react with the protein overnight. The enzyme has apparently low activity on whey proteins, but still sufficient to affect viscosity development upon heating, as demonstrated in this example. It is clear that the lysyl oxidases are capable to generate reactive protein intermediates that can be used in a second step by heating to form modify the texture of concentrated whey solutions. Whey proteins are present as texturizing agents in many food stuffs; it is of industrial interest to be able to modify the texturizing properties of whey proteins with lysyl oxidase, and it is of special interest to be able to prepare reactive intermediates that can subsequently be used to modify food texture.

### Example 9

### Use of lysyl oxidase ZGR to improve the volume of rice bread

480 grams of rice flour (BL-250, van Sillevoldt, Netherlands) and 20 grams of rice protein (Remy Industries, 0400061) were mixed in a Hobart mixer (first speed: 2 minutes, second speed:3 minutes) with 475 grams of water, 22.5 grams of Koningsgist (Gilde, The Netherlands), 10 grams NaCl, 5 grams sucrose, 50 grams biskien (fat, UniPro, Netherlands), 20 grams of xanthan gum and 12 ppm Bakezyme P500 (DSM, Netherlands). Doughs were moulded by hand, first proof for 20 minutes at 38°C, final proof for 40 minutes at 38°C (both at 85% relative humidity). Lysyl oxidase ZGR (100 ppm) was added before mixing in one experiment, in the control experiment no lysyl oxidase was added. The dough with the lysyl oxidase was judged more developed. Breads were baked (240°C, 20 minutes) using a MIWE Combo oven (Arnstein, Germany, closed valve). After baking, the bread containing the lysyl oxidase was more stable in structure and higher in volume.

### Example 10

### Cross linking of milk proteins

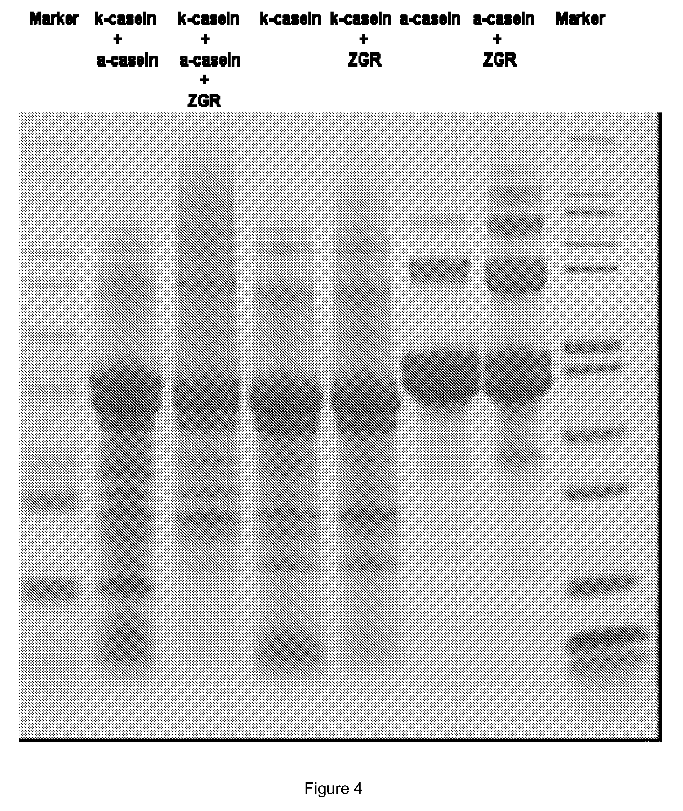

3% (w/v) solutions were prepared in water of κ-casein, α-casein and a mixture of κ- and α-casein (both 0.3% w/v) and incubated overnight at 37°C with lysyl oxidase ZGR (0.1 mg/ml). Subsequently, samples were heated at 85°C (30 minutes). Control samples were prepared in which lysyl oxidase ZGR was not added. Samples were analyzed on SDS-PAGE (see Figure 4). Clearly, the addition of the lysyl oxidase results in the formation of proteins with increased molecular weight, as compared to the control in which lysyl oxidase was not added. The treatment with lysyl oxidase ZGR leads to covalent protein cross links, leading to the higher molecular weight aggregates.

## Claims

1. Use of a polypeptide which has lysyl oxidase activity and which comprises the following amino acid motif, wherein residues in brackets indicate allowed degeneracy at that point, and amino acids are in 1-letter code:
- IHD[NS]LSGSMHDHV[IL]NFK,
to catalyze the oxidative deamination of lysyl groups in a protein or peptide to the corresponding aldehydes, with the subsequent reduction of O₂ to H₂O₂.

2. Use of the polypeptide according to claim 1 whereby the polypeptide has an amino acid sequence which has at least 60% amino acid sequence identity with the whole amino acid sequence of any one of SEQ ID NO: 1 to 4.

3. Use of the polypeptide according to claim 2 which has an amino acid sequence which has at least 65%, preferably at least 70%, more preferably at least 80%, even more preferably at least 90%, most preferably at least 95%, and even most preferably at least about 97% identity with the whole amino acid sequence of any one of SEQ ID NO: 1 to 4.

4. Use of the polypeptide according to claim 2, comprising any one of the amino acid sequences of SEQ ID NO: 1 to 4.

5. The use of the polypeptide according to claim 2 or the use of the lysyl oxidase according to claim 1, whereby the polypeptide or lysyl oxidase is obtained from a fungus, preferably an *Aspergillus,* more preferably from *Aspergillus nidulans, Cryptococcus neoformans, Podospora anserine* and *Fusarium graminearum.*

6. The use of the polypeptide according to claim 2 or the use of the lysyl oxidase according to claim 1 for the preparation of a food or feed or a nutraceutical or for the preparation of an intermediate product for the preparation of a food or feed or a nutraceutical.

7. A method for the production of lysyl oxidase which comprises cultivating a host cell comprising a nucleic acid construct comprising a polynucleotide encoding the lysyl oxidase which comprises the amino acid sequence:
- IHD[NS]LSGSMHDHV[IL]NFK;
under conditions suitable for production of the lysyl oxidase; and recovering the lysyl oxidase.

8. A process for identifying new lysyl oxidases comprising screening an amino acid sequence for the presence of the amino acid sequences:
- IHD[NS]LSGSMHDHV[IL]NFK
and testing the identified lysyl oxidases for lysyl oxidase activity.

9. A process to modify a food or feed product which contains a protein, said method comprising modifying the food or feed product by contacting it with a lysyl oxidase which comprises the following amino acid motif, wherein residues in brackets indicate allowed degeneracy at that point, and amino acids are in 1-letter code:
- IHD[NS]LSGSMHDHV[IL]NFK.

10. A process for the preparation of a reactive aldehyde in a protein or peptide which comprises the oxidative deamination of lysyl-amine of the protein or peptide by a lysyl oxidase and preferably concentrating and/or drying the protein or peptide containing the reactive aldehydes, whereby the lysyl oxidase comprises the following amino acid motif wherein residues in brackets indicate allowed degeneracy at that point, and amino acids are in 1-letter code:
- IHD[NS]LSGSMHDHV[IL]NFK.

11. A process for the preparation of a cross-linked protein or peptide which comprises heating a protein or peptide which comprises reactive aldehydes and prepared according to the process of claim 10, to cross-link the protein.

## Patentansprüche

1. Verwendung eines Polypeptids, das Lysyloxidaseaktivität aufweist und das das folgende Aminosäuremotiv umfasst, wobei Reste in Klammern eine erlaubte Degeneration an jenem Punkt anzeigen und Aminosäuren im Einbuchstaben-Code angegeben sind:
- IHD[NS]LSGSMHDHV[IL]NFK,
zum Katalysieren der oxidativen Deaminierung von Lysylgruppen in einem Protein oder Peptid zu den entsprechenden Aldehyden, mit der anschließenden Reduktion von O₂ zu H₂O₂.

2. Verwendung des Polypeptids nach Anspruch 1, wobei das Polypeptid eine Aminosäuresequenz aufweist, die über mindestens 60% Aminosäuresequenzidentität zu der gesamten Aminosäuresequenz von einer der SEQ ID NO: 1 bis 4 verfügt.

3. Verwendung des Polypeptids nach Anspruch 2, das eine Aminosäuresequenz aufweist, die über mindestens 65%, vorzugsweise mindestens 70%, stärker bevorzugt mindestens 80%, noch stärker bevorzugt mindestens 90%, am stärksten bevorzugt mindestens 95% und am allerstärksten bevorzugt mindestens 97% Identität zu der gesamten Aminosäuresequenz von einer der SEQ ID NO: 1 bis 4 verfügt.

4. Verwendung des Polypeptids nach Anspruch 2, das eine beliebige der Aminosäuresequenzen von SEQ ID NO: 1 bis 4 umfasst.

5. Verwendung des Polypeptids nach Anspruch 2 oder Verwendung der Lysyloxidase nach Anspruch 1, wobei das Polypeptid oder die Lysyloxidase von einem Pilz, vorzugsweise einem *Aspergillus,* stärker bevorzugt von *Aspergillus nidulans, Cryptococcus neoformans, Podospora anserine* und *Fusarium graminearum,* erhalten wird.

6. Verwendung des Polypeptids nach Anspruch 2 oder Verwendung der Lysyloxidase nach Anspruch 1 zur Herstellung eines Nahrungsmittels oder Futtermittels oder eines Nutrazeutikums oder zur Herstellung eines Zwischenprodukts für die Herstellung eines Nahrungsmittels oder Futtermittels oder Nutrazeutikums.

7. Verfahren zur Produktion von Lysyloxidase, bei dem man eine Wirtszelle umfassend ein Nukleinsäurekonstrukt umfassend ein Polynukleotid, das für die Lysyloxidase, die die Aminosäuresequenz:
- IHD[NS]LSGSMHDHV[IL]NFK
umfasst, codiert, unter Bedingungen kultiviert, die sich für die Produktion der Lysyloxidase eignen; und die Lysyloxidase gewinnt.

8. Verfahren zum Identifizieren von neuen Lysyloxidasen, bei dem man eine Aminosäuresequenz auf das Vorhandensein der Aminosäuresequenzen:
- IHD[NS]LSGSMHDHV[IL]NFK
durchmustert und die identifizierten Lysyloxidasen auf Lysyloxidaseaktivität testet.

9. Verfahren zum Modifizieren eines Nahrungsmitteloder Futtermittelprodukts, das ein Protein enthält, wobei man bei dem Verfahren das Nahrungsmittel- oder Futtermittelprodukt dadurch modifiziert, dass man es mit einer Lysyloxidase, die das folgende Aminosäuremotiv enthält, in Kontakt bringt, wobei Reste in Klammern eine erlaubte Degeneration an jenem Punkt anzeigen und Aminosäuren im Einbuchstaben-Code angegeben sind:
- IHD[NS]LSGSMHDHV[IL]NFK.

10. Verfahren zur Herstellung eines reaktionsfähigen Aldehyds in einem Protein oder Peptid, wobei man Lysylamin des Proteins oder Peptids mit einer Lysyloxidase oxidativ deaminiert und vorzugsweise das Protein oder Peptid, das die reaktionsfähigen Aldehyde enthält, konzentriert und/oder trocknet, wobei die Lysyloxidase das folgende Aminosäuremotiv umfasst, wobei Reste in Klammern eine erlaubte Degeneration an jenem Punkt anzeigen und Aminosäuren im Einbuchstaben-Code angegeben sind:
- IHD[NS]LSGSMHDHV[IL]NFK.

11. Verfahren zur Herstellung eines vernetzten Proteins oder Peptids, bei dem man ein Protein oder Peptid, das reaktionsfähige Aldehyde umfasst, die nach dem Verfahren von Anspruch 10 hergestellt wurden, erhitzt, um das Protein zu vernetzen.

## Revendications

1. Utilisation d'un polypeptide qui a une activité de lysyl oxydase et qui comprend le motif d'acides aminés suivant, dans lequel
les résidus entre parenthèses indiquent une dégénérescence autorisée en ce point, et les acides aminés sont en un code à 1 lettre :
- IHD[NS]LSGSMHDHV[IL]NFK,
pour catalyser la désamination oxydative de groupes lysyle dans une protéine ou un peptide en les aldéhydes correspondants, avec la réduction ultérieure du O₂ en H₂O₂.

2. Utilisation du polypeptide selon la revendication 1, moyennant quoi le polypeptide a une séquence d'acides aminés qui a une identité de séquence d'acides aminés d'au moins 60% avec la séquence d'acides aminés entière de l'une quelconque des SEQ ID n° : 1 à 4.

3. Utilisation du polypeptide, selon la revendication 2, qui a une séquence d'acides aminés ayant une identité d'au moins 65%, de préférence d'au moins 70%, mieux préféré d'au moins 80%, même mieux préféré d'au moins 90%, de manière préférée entre toutes d'au moins 95% et, même de manière préférée entre toutes, d'au moins 97% avec la séquence d'acides aminés entière de l'une quelconque des SEQ ID n° : 1 à 4.

4. Utilisation du polypeptide, selon la revendication 2, comprenant l'une quelconque des séquences d'acides aminés de SEQ ID n° : 1 à 4.

5. Utilisation du polypeptide, selon la revendication 2, ou utilisation de la lysyl oxydase selon la revendication 1, moyennant quoi le polypeptide ou la lysyl oxydase est obtenu(e) à partir d'un champignon, de préférence d'un *Aspergillus,* mieux préféré à partir *d'Aspergillus nidulans, Cryptococcus neoformans, Podospora anserine* et *Fusarium graminearum.*

6. Utilisation du polypeptide selon la revendication 2, ou utilisation de la lysyl oxydase selon la revendication 1, pour la préparation d'un aliment, d'un aliment pour animaux ou d'un produit nutraceutique ou pour la préparation d'un produit intermédiaire destiné à la préparation d'un aliment, d'un aliment pour animaux ou d'un produit nutraceutique.

7. Procédé de production d'une lysyl oxydase qui comprend la culture d'une cellule hôte, comprenant un construit d'acide nucléique comprenant un polynucléotide qui code pour la lysyl oxydase, qui comprend la séquence d'acides aminés :
- IHD[NS]LSGSMHDHV[IL]NFK ;
dans des conditions appropriées pour la production de la lysyl oxydase ; et la récupération de la lysyl oxydase.

8. Processus d'identification de nouvelles lysyl oxydases comprenant le criblage d'une séquence d'acides aminés en vue de la présence des séquences d'acides aminés :
- IHD[NS]LSGSMHDHV[IL]NFK
ainsi que le test des lysyl oxydases identifiées en vue d'une activité de lysyl oxydase.

9. Processus de modification d'un produit alimentaire ou d'alimentation animale qui contient une protéine, ledit procédé comprenant la modification du produit alimentaire ou d'alimentation animale en le mettant en contact avec une lysyl oxydase, qui comprend le motif d'acides aminés suivant, dans lequel
les résidus entre parenthèses indiquent une dégénérescence autorisée en ce point, et les acides aminés sont en un code à 1 lettre :
- IHD[NS]LSGSMHDHV[IL]NFK.

10. Processus de préparation d'un aldéhyde réactif dans une protéine ou un peptide, qui comprend la désamination oxydative d'une lysyl-amine de la protéine ou du peptide, par une lysyl oxydase, et de préférence, la concentration et/ou le séchage de la protéine ou du peptide contenant les aldéhydes réactifs, moyennant quoi la lysyl oxydase comprend le motif d'acides aminés suivant, dans lequel les résidus
entre parenthèses indiquent une dégénérescence autorisée en ce point, et les acides aminés sont en un code à 1 lettre :
- IHD[NS]LSGSMHDHV[IL]NFK.

11. Processus de préparation d'une protéine ou d'un peptide réticulé(e) qui comprend le chauffage d'une protéine ou d'un peptide, qui comprend des aldéhydes réactifs et étant préparés selon le processus de la revendication 10, pour réticuler la protéine.
